# FASCICULE DE BREVET EUROPEEN

(11) **EP 4 416 291 B1**
(45) Date de publication et mention de la délivrance du brevet: **21.01.2026**
(21) Numéro de dépôt: 22802031.9
(22) Date de dépôt: 07.10.2022
(51) Int. Cl.: C12N 15/62, C07K 14/395, C07K 1/22

(54) **PROCÉDÉ DE PURIFICATION D'UNE PROTÉINE D'INTÉRÊT ET MOYENS POUR SA MISE EN OEUVRE**
VERFAHREN ZUR REINIGUNG EINES PROTEINS VON INTERESSE UND MITTEL ZUR DURCHFÜHRUNG DES VERFAHRENS
METHOD FOR PURIFYING A PROTEIN OF INTEREST AND MEANS FOR IMPLEMENTING SAME

(30) Priorité: 12.10.2021 FR 2110762
(43) Date de publication de la demande: 21.08.2024
(73) Titulaire: Université Grenoble Alpes, 38400 Saint Martin d'Hères (FR); Centre National de la Recherche Scientifique, 75016 Paris (FR); Institut National de la Santé et de la Recherche Médicale, 75013 Paris (FR)
(72) Inventeur: VERDEL, André, 38330 BIVIERS (FR); TODESCHINI, Leïla, 38700 LE SAPPEY EN CHARTREUSE (FR); PERAZZA, Daniel, 38650 SINARD (FR)
(74) Mandataire: Germain Maureau
(86) Numéro de dépôt international: PCT/EP2022/077890
(87) Numéro de publication internationale: WO 2023/061859

(56) Documents cités:
- WO-A1-2017/194888
- SHICHINO YUICHI ET AL: "Meiotic gene silencing complex MTREC/NURS recruits the nuclear exosome to YTH-RNA-binding protein Mmi1", vol. 16, no. 2, 3 February 2020 (2020-02-03), USA, pages e1008598, XP055919569, ISSN: 1553-7390, Retrieved from the Internet <URL:https://journals.plos.org/plosgenetics/> DOI: 10.1371/journal.pgen.1008598
- XIE GUODONG ET AL: "A conserved dimer interface connects ERH and YTH family proteins to promote gene silencing", vol. 10, no. 1, 1 December 2019 (2019-12-01), XP055919578, Retrieved from the Internet <URL:https://www.nature.com/articles/s41467-018-08273-9.pdf> DOI: 10.1038/s41467-018-08273-9
- HIRIART EDWIGE ET AL: "Mmi1 RNA surveillance machinery directs RNAi complex RITS to specific meiotic genes in fission yeast : Targeting RITS to meiotic RNAs and genes", THE EMBO JOURNAL / EUROPEAN MOLECULAR BIOLOGY ORGANIZATION, vol. 31, no. 10, 16 May 2012 (2012-05-16), Oxford, pages 2296 - 2308, XP055919717, ISSN: 0261-4189, DOI: 10.1038/emboj.2012.105
- WU BAIXING ET AL: "Structural insights into the specific recognition of DSR by the YTH domain containing protein Mmi1", vol. 491, no. 2, 1 September 2017 (2017-09-01), Amsterdam NL, pages 310 - 316, XP055919712, ISSN: 0006-291X, Retrieved from the Internet <URL:https://www.sciencedirect.com/science/article/pii/S0006291X17314559/pdfft?md5=f0c71b8ba594b1e0ada4c40d28cd3360&pid=1-s2.0-S0006291X17314559-main.pdf> DOI: 10.1016/j.bbrc.2017.07.104
- STOWELL JAMES A.W. ET AL: "A low-complexity region in the YTH domain protein Mmi1 enhances RNA binding", vol. 293, no. 24, 1 June 2018 (2018-06-01), US, pages 9210 - 9222, XP055919724, ISSN: 0021-9258, Retrieved from the Internet <URL:https://www.ncbi.nlm.nih.gov/pmc/articles/PMC6005420/pdf/zbc9210.pdf> DOI: 10.1074/jbc.RA118.002291
- WANG CHONGYUAN ET AL: "A novel RNA-binding mode of the YTH domain reveals the mechanism for recognition of determinant of selective removal by Mmi1", vol. 44, no. 2, 29 January 2016 (2016-01-29), GB, pages 969 - 982, XP055919560, ISSN: 0305-1048, Retrieved from the Internet <URL:https://academic.oup.com/nar/article-pdf/44/2/969/17438621/gkv1382.pdf> DOI: 10.1093/nar/gkv1382

## Description

La présente invention s'inscrit dans le domaine de la purification par affinité des protéines d'intérêt.

Plus particulièrement, la présente invention concerne un procédé de purification d'une protéine d'intérêt. D'autres objets de l'invention sont un support solide et un kit pour la mise en œuvre d'un procédé de purification selon l'invention.

La production en grande quantité, et à faible coût, de protéines d'intérêt s'avère d'un intérêt croissant dans de nombreux secteurs de l'industrie biotechnologique.

A l'heure actuelle, les protéines sont principalement produites par culture de lignées cellulaires spécialement conçues pour les exprimer à partir des gènes codant pour ces protéines, qui sont intégrés dans ces lignées cellulaires. Si de telles méthodes de production par voie biologique permettent de produire les protéines d'intérêt efficacement et en grande quantité, ces dernières sont cependant obtenues au sein de mélanges complexes, contenant notamment les éléments nécessaires à la culture des lignées cellulaires, ainsi que les autres composants cellulaires des cellules. Il est de ce fait nécessaire d'isoler les protéines d'intérêt produites de ces mélanges complexes, afin de les récupérer sous une forme suffisamment pure pour les applications visées, ceci devant être réalisé sans en compromettre l'activité biologique nécessaire à ces applications. Cet objectif est particulièrement crucial lorsque les protéines d'intérêt sont, par exemple, des hormones, des peptides antibiotiques, des régulateurs d'enzymes, etc., ou toute autre protéine destinée à une application thérapeutique.

De nombreux procédés ont été proposés par l'art antérieur pour la purification des protéines d'intérêt produites par des procédés biologiques.

Nombre de ces procédés consistent à exprimer la protéine d'intérêt sous forme d'une protéine de fusion au sein de laquelle elle est associée, par les techniques de génie génétique, à une étiquette protéique présentant une affinité particulière pour un partenaire. Il est alors possible de purifier la protéine d'intérêt par chromatographie d'affinité, le partenaire de l'étiquette protéique étant greffé sur le support de chromatographie.

Au titre de telles étiquettes protéiques proposées par l'art antérieur, on peut notamment citer l'étiquette histidine, présentant une haute affinité pour les cations cobalt et nickel, l'étiquette Glutathion S-transférase, présentant une haute affinité pour le glutathion, et la protéine liant le maltose (MBP), à haute affinité pour le maltose.

A titre d'autre exemple, le document WO 2017/194888 décrit un procédé de purification de protéines d'intérêt par affinité basée sur l'activité lectinique du domaine CRD d'une galectine.

Aucun des procédés proposés par l'art antérieur ne permet cependant de produire et purifier une protéine d'intérêt à la fois à bas coût, rapidement, et avec un haut rendement et une haute spécificité menant à l'obtention de la protéine d'intérêt avec un taux de pureté élevé. La présente invention vise à proposer un tel procédé.

A cet effet, la présente invention met à profit la forte capacité d'un domaine particulier de la protéine Mmi1 des espèces du genre *Schizosaccharomyces à* se lier à une molécule d'acide ribonucléique (ARN) de séquence particulière, ceci avec une haute spécificité.

La protéine Mmi1 (pour l'anglais Meiotic mRNA interception protein 1), joue au sein des cellules un rôle important dans un évènement post-transcriptionnel particulier, l'élimination sélective des ARN messagers spécifiques de la méiose. Il a été décrit dans la littérature que cette protéine se lie avec une haute spécificité de liaison à l'ARN, plus particulièrement à une séquence contenant des répétitions de l'hexanucléotide UNAAAC (E. Hiriart et al., The Embo Journal, 2012, 31(10), 2296-2308 ; Wu et al., Biochemical and Biophysical Research Communications, 2017, 491, 310-316). Cette affinité a été plus particulièrement attribuée au domaine nommé YTH (pour l'anglais YT521-B homology) de Mmi1 de *Schizosaccharomyces,* localisé dans la région C-terminale de la protéine (Stowell et al., J. Biol. Chem., 2018, 293(24), 9210-9222).

La publication de Shichino Yuichi et al., dans Plos Genetics, 2020, 16(2):e1008598 décrit une protéine de fusion entre rrp6, GFP ou YFP et la protéine Mmi1 de *Schizosaccharomyces pombe* entière ou tronquée. La publication de Xie Guodong et al., dans Nature Communications, 2019, 10:251 décrit une protéine de fusion entre Erh1 ou GST et Mmi1 de *Schizosaccharomyces pombe* entière ou tronquée.

De manière tout à fait surprenante, il a été découvert par les présents inventeurs que non seulement la fusion d'une protéine Mmi1 des espèces du genre *Schizosaccharomyces,* ou d'un de ses fragments comprenant au moins son domaine YTH élargi du côté N-terminal et du côté C-terminal (ce domaine YTH élargi, constitué par les 173 acides aminés C-terminaux de la protéine Mmi1, étant désigné dans la présente description, pour plus de commodité, par l'abréviation « YTH+ »), à une protéine d'intérêt, n'impacte pas la capacité de cette protéine Mmi1 ou de ce fragment à se lier spécifiquement à la séquence d'ARN à motif UNAAAC mentionnée ci-dessus, et ceci que la fusion soit réalisée en N-terminal ou en C-terminal de la protéine Mmi1 ou de son fragment ; mais qu'en outre, la mise en présence d'une molécule d'acide ribonucléique contenant au moins un motif de séquence nucléotidique UNAAAC avec un extrait de culture cellulaire contenant une protéine de fusion « protéine d'intérêt / protéine Mmi1 ou fragment de cette dernière contenant le domaine YTH+ », ne provoque pas de dégradation massive de cette molécule d'ARN dans cet extrait de culture cellulaire. Ainsi, alors que l'homme du métier n'aurait jamais envisagé de mettre en œuvre, dans un procédé de purification d'une protéine d'intérêt produite par un procédé biologique, une étape de mise en présence du milieu contenant la protéine à purifier avec une molécule d'ARN en tant que partenaire d'une liaison par affinité avec l'étiquette fusionnée à la protéine d'intérêt, il a été découvert par les présents inventeurs que non seulement un tel procédé est tout à fait réalisable pour le cas particulier du couple « Mmi1 de *Schizosaccharomyces* ou fragment de cette dernière contenant le domaine YTH+ / molécule d'ARN à séquence UNAAAC », mais qu'il s'avère en plus hautement performant, puisqu'il permet de purifier la protéine d'intérêt avec une haute spécificité et un haut rendement, en un temps court, sans altérer les fonctions de la protéine d'intérêt. Ainsi, selon un premier aspect, il est proposé par la présente invention un procédé de purification d'une protéine d'intérêt, qui comprend :
- la préparation d'une protéine de fusion comprenant la protéine d'intérêt fusionnée à une étiquette protéique, ladite étiquette protéique comprenant au moins, ou étant constituée de : la protéine Mmi1 d'un microorganisme du genre *Schizosaccharomyces,* un fragment de cette protéine Mmi1 en comprenant au moins les 173 acides aminés C-terminaux (le domaine de la protéine Mmi1 formé par ces 173 acides aminés C-terminaux étant désigné dans la présente description par l'abréviation YTH+), ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC,
- la mise en présence de cette protéine de fusion avec une molécule d'acide ribonucléique contenant au moins un motif de séquence nucléotidique UNAAAC, de sorte à permettre la liaison par affinité de l'étiquette protéique avec cette molécule d'acide ribonucléique, plus précisément avec son motif de séquence nucléotidique UNAAAC, cette molécule d'acide ribonucléique étant greffée sur un support solide ou couplée à un ligand de capture,
- le cas échéant, lorsque ladite molécule d'acide ribonucléique est couplée à un ligand de capture, la mise en présence de cette molécule d'acide ribonucléique, à laquelle est liée la protéine d'intérêt par l'intermédiaire de l'étiquette protéique, avec un partenaire d'affinité du ligand de capture greffé sur un support solide,
- optionnellement, l'isolement du support solide du milieu dans lequel il est contenu, par exemple un milieu de culture cellulaire dans lequel les cellules exprimant la protéine de fusion ont été cultivées, et/ou un milieu de lyse cellulaire de telles cellules,
- et la séparation de la protéine d'intérêt, seule ou au sein de la protéine de fusion qui la contient, et du support solide, pour récupérer la protéine d'intérêt.

On entend dans la présente description, par ligand de capture, une molécule qui est d'une part apte à être couplée de manière covalente avec une molécule d'acide ribonucléique, et d'autre part capable se lier avec une forte affinité et spécificité à un partenaire d'affinité, par exemple un récepteur protéique, celui-ci pouvant quant à lui être fixé sur un support solide. A titre d'exemple de tel ligand de capture utilisable selon l'invention, on peut citer la biotine, dont des partenaires d'affinité sont l'avidine et la streptavidine.

Le procédé selon l'invention permet avantageusement, à lui seul, de produire la protéine d'intérêt, sous forme d'une protéine de fusion recombinante, et de la séparer du milieu de production en tirant avantage de la forte capacité de liaison hautement spécifique de l'étiquette protéique comprenant le domaine YTH+ d'une protéine Mmi1 d'une espèce du genre de *Schizosaccharomyces* avec la séquence d'ARN UNAAAC, pour obtenir la protéine d'intérêt rapidement avec un haut degré de pureté et un haut rendement. Ces étapes peuvent avantageusement être réalisées facilement et rapidement, et à bas coût.

De manière conventionnelle, dans la séquence nucléotidique UNAAAC, U désigne l'uracile, A désigne l'adénine, C désigne la cytosine et N désigne toute base parmi l'adénine, la cytosine, la guanine et l'uracile. Cette séquence nucléotidique est ici représentée, comme toutes les autres séquences nucléotidiques décrites, de manière conventionnelle, c'est-à-dire dans le sens de l'extrémité 5' vers l'extrémité 3' (les séquences d'acides aminés étant quant à elles représentées, de manière également conventionnelle, dans le sens de lecture de l'extrémité N-terminale vers l'extrémité C-terminale).

La molécule d'acide ribonucléique contenant le motif UNAAAC peut comprendre un seul exemplaire de ce motif, ou une ou plusieurs répétitions de ce motif. Elle peut en outre comprendre, en 5' ou en 3' de ce motif UNAAAC ou de cette série de motifs UNAAAC, un ou plusieurs acides ribonucléiques supplémentaires, par exemple 2 à 10, en particulier 2 à 6, acides ribonucléiques supplémentaires.

Le greffage / couplage de la molécule d'acide ribonucléique au support solide / au ligand de capture peut être réalisé par toute méthode classique en elle-même. Ce greffage / couplage est de préférence réalisé par liaison covalente, préférentiellement au niveau de l'extrémité 5' ou à l'extrémité 3' de la molécule d'acide ribonucléique.

On entend dans la présente description, par protéine d'intérêt, toute protéine, peptide ou polypeptide, sous forme native ou recombinante, présentant un intérêt pour une application visée, en particulier pour une application comprenant une administration à un mammifère, notamment un humain.

Le procédé selon l'invention peut par exemple avantageusement être utilisé pour la purification de complexes endogènes dans le domaine de la recherche fondamentale, d'hormones, de peptides antibiotiques ou encore de régulateurs d'enzymes dans le domaine de la recherche appliquée, une telle liste n'étant nullement limitative de l'invention.

L'étiquette protéique mise en œuvre dans le procédé selon l'invention peut comprendre la protéine Mmi1 d'un microorganisme d'une espèce du genre *Schizosaccharomyces* entière ou un de ses fragments en contenant au moins le domaine YTH+ (c'est-à-dire les 173 acides aminés C-terminaux).

La protéine Mmi1 est de préférence issue d'une espèce choisie parmi *Schizosaccharomyces pombe, Schizosaccharomyces japonicus, Schizosaccharomyces octosporus* et *Schizosaccharomyces cryophilus.*

Il est du ressort de l'homme du métier d'identifier, pour une espèce du genre *Schizosaccharomyces* donnée, la séquence d'acide aminés de la protéine Mmi1, ainsi, le cas échéant, que la séquence du gène codant pour cette protéine. De telles données sont notamment accessibles dans les bases de données de séquences protéiques et de séquences nucléotidiques. A titre d'exemple, dans la base de données GenBank, les séquences d'acides aminés de la protéine Mmi1 sont accessibles, pour l'espèce *Schizosaccharomyces pombe,* sous le numéro d'accession NP_587783.2 (SEQ ID No : 1 - le gène codant pour cette protéine de cette espèce a pour séquence la séquence SEQ ID No : 2), pour l'espèce *Schizosaccharomyces japonicus,* sous le numéro d'accession XP_002173827.2 (SEQ ID No : 3 - le gène codant pour cette protéine de cette espèce a pour séquence la séquence SEQ ID No : 4), pour l'espèce *Schizosaccharomyces octosporus,* sous le numéro d'accession XP_013019124.1 (SEQ ID No : 5 - le gène codant pour cette protéine de cette espèce a pour séquence la séquence SEQ ID No : 6), et pour l'espèce *Schizosaccharomyces cryophilus,* sous le numéro d'accession XP_013025346.1 (SEQ ID No : 7 - le gène codant pour cette protéine de cette espèce a pour séquence la séquence SEQ ID No : 8).

Lorsque le microorganisme du genre *Schizosaccharomyces* appartient à l'espèce *Schizosaccharomyces pombe,* le domaine YTH+ de la protéine Mmi1 s'étend du résidu en position 316 (résidu leucine) au résidu en position 488 (résidu arginine, en position C-terminale dans la séquence de la protéine). Le domaine YTH+ a alors pour séquence d'acides aminés la séquence SEQ ID No : 9.

Lorsque le microorganisme du genre *Schizosaccharomyces* appartient à l'une des espèces *Schizosaccharomyces japonicus, Schizosaccharomyces octosporus* et *Schizosaccharomyces cryophilus,* le fragment de la protéine Mmi1 contient de préférence les 174 acides aminés C-terminaux de la protéine. Le fragment de la protéine Mmi1 utilisé selon l'invention contient ainsi de préférence, ou consiste en :
- pour l'espèce *Schizosaccharomyces japonicus,* le domaine de la protéine Mmi1 s'étendant du résidu en position 306 (résidu leucine) au résidu en position 479 (résidu arginine, en position C-terminale dans la séquence de la protéine). Le domaine a alors pour séquence d'acides aminés la séquence SEQ ID No : 10 ;
- pour l'espèce *Schizosaccharomyces octosporus,* le domaine de la protéine Mmi1 s'étendant du résidu en position 307 (résidu leucine) au résidu en position 480 (résidu arginine, en position C-terminale dans la séquence de la protéine). Le domaine a alors pour séquence d'acides aminés la séquence SEQ ID No : 11 ;
- pour l'espèce *Schizosaccharomyces cryophilus,* le domaine de la protéine Mmi1 s'étendant du résidu en position 306 (résidu leucine) au résidu en position 479 (résidu arginine, en position C-terminale dans la séquence de la protéine). Le domaine a alors pour séquence d'acides aminés la séquence SEQ ID No : 12.

Ainsi, dans des modes de mise en œuvre particuliers de l'invention, le fragment de la protéine Mmi1 en comprenant au moins les 173 acides aminés C-terminaux présente une séquence d'acides aminés comprenant, ou consistant en, la séquence d'acides aminés SEQ ID No : 9 (correspondant à *Schizosaccharomyces pombe*)*,* SEQ ID No : 10 (correspondant à *Schizosaccharomyces japonicus*), SEQ ID No : 11 (correspondant à *Schizosaccharomyces octosporus*) ou SEQ ID No : 12 (correspondant à *Schizosaccharomyces cryophilus*) :
- SEQ ID No : 9 : 
LNFSRASEHRNEKGERISMINPRVVLDENGISHRSRYFIMLCDNETAIAHAKKT
SIWAVKKDSSKRISDAYKKASVYFIFVAQQTYNALGYAQVVSDLNSTELPFWS
DSSHAGGVRIKWIKTCNLFSAEISEIVSHMDHGSEARDGMEMMYDEGSRLCT
LINYAIMKRIGRDR
- SEQ ID No : 10 : 
LDFEHAHEYRNEKGERVSMINPRVILDENGISHRSRYFIMLVDNETAISHAKRS
SVWSLKQNYANAISEAYKQEANVYFIFINAKSYNALGYAQLTSDVGTVAKPFW
ADTTYTNGVNVKWIKTCNLYSNEISEIVSRMDHGARARDGQEMMYDEGSRL
 CTLINSAIMKRIGRDR
- SEQ ID No : 11 : 
LNFARASEYRNDKGERISMINPRVILDENGISSRSRYFIMLCDNETAISHAKKT
SIWAVKHDAASRVSDAYKNASIYFIFIAKPTNNALGYAQVVSDLNSAELPFWA
DNATYAGGVRVKWIKTCNLFSAEISDIVGRMNHGATARDGMEMMYDEGCRL
CILVNSAIMKRIGRDR
- SEQ ID No : 12 :

LNFARASEYRNDKGERISMINPRVILDESGISHRSRYFIMLCDNETAIAHAKKT
SIWAVKHEAASRVSDAYKKASIYFIFIAKPTNNALGYAQVVSDLNSAELPFWAD
NATYAGGVRVKWIKTCNLFSAEISDIVGRMNHGMTAKDGMEMMYDEGCRLCI
LVNSAIMKRIGRDR

L'étiquette protéique mise en œuvre selon l'invention peut aussi bien comprendre, ou consister en, la protéine Mmi1 ou un fragment de la protéine Mmi1 contenant au moins le domaine YTH+ de la protéine, qu'une protéine de séquence d'acides aminés ayant au moins 90 %, de préférence au moins 95 %, préférentiellement au moins 98 % et préférentiellement encore au moins 99 %, d'identité avec la séquence d'acides aminés de la protéine Mmi1 ou dudit fragment de ladite protéine Mmi1, et capable de se lier à un motif d'acide ribonucléique de séquence nucléotidique UNAAAC, de préférence avec une spécificité au moins aussi bonne que le domaine YTH+ de la protéine.

Pour toute protéine de séquence d'acides aminés donnée ayant au moins 90 %, de préférence au moins 95 %, préférentiellement au moins 98 % et préférentiellement encore au moins 99 %, d'identité avec la séquence d'acides aminés de la protéine Mmi1 ou dudit fragment de la protéine Mmi1, il entre dans les compétences de l'homme du métier d'évaluer sa capacité de liaison au motif d'ARN de séquence UNAAAC, par des tests de liaison classiques en eux-mêmes, par exemple par des tests de retard sur gel après électrophorèse ou de spectroscopie à fluorescence, de dichroïsme circulaire ou de résonnance plasmonique. La spécificité de liaison peut être évaluée par ces mêmes méthodes, par comparaison avec de molécules d'ARN de séquence proche de la séquence UNAAAC (par exemple la séquence CNAAAC ou GNAAAC) et de comparer les résultats obtenus avec ceux obtenus avec ladite protéine Mmi1 ou ledit fragment de la protéine Mmi1.

La protéine de séquence d'acides aminés ayant au moins 90 %, de préférence au moins 95 %, préférentiellement au moins 98 % et préférentiellement encore au moins 99 %, d'identité avec la séquence d'acides aminés de la protéine Mmi1 ou dudit fragment de la protéine Mmi1 peut présenter, par rapport à la séquence de la protéine Mmi1 ou dudit fragment de la protéine Mmi1, qui constitue la séquence de référence, des insertions, délétions et/ou substitutions. Dans le cas d'une substitution, celle-ci est de préférence réalisée par un acide aminé de la même famille que l'acide aminé d'origine, par exemple par substitution d'un résidu basique tel que l'arginine par un autre résidu basique comme un résidu lysine, d'un résidu acide tel que l'aspartate par un autre résidu acide comme le glutamate, d'un résidu polaire comme la sérine par un autre résidu polaire comme la thréonine, d'un résidu aliphatique comme la leucine par un autre résidu aliphatique comme l'isoleucine, etc.

Le pourcentage d'identité entre deux séquences d'acides aminés est ici déterminé de manière classique en elle-même, en comparant les deux séquences alignées de manière optimale, à travers une fenêtre de comparaison, la partie de la séquence d'acides aminés à comparer située dans la fenêtre de comparaison pouvant comprendre des additions ou des délétions par rapport à la séquence de référence de sorte à obtenir un alignement optimal entre les deux séquences. Le pourcentage d'identité est ensuite calculé en déterminant le nombre de positions pour lesquelles un résidu d'acide aminé est identique dans les deux séquences comparées, puis en divisant ce nombre de positions par le nombre total de positions dans la fenêtre de comparaison, le nombre obtenu étant multiplié par cent pour obtenir le pourcentage d'identité entre les deux séquences.

Le procédé selon l'invention peut en outre répondre à l'une ou plusieurs des caractéristiques décrites ci-après, mises en œuvre isolément ou en chacune de leurs combinaisons techniquement opérantes.

Au sein de la protéine de fusion, l'étiquette protéique peut être fusionnée à l'extrémité N-terminale ou à l'extrémité C-terminale de la protéine d'intérêt.

Le cas échéant, un espaceur peut être intégré entre la protéine d'intérêt et l'étiquette protéique.

Dans des modes de mise en œuvre particuliers de l'invention, un site de clivage enzymatique est inséré entre la protéine d'intérêt et l'étiquette protéique. Tout site de clivage classique en lui-même entre dans le cadre de l'invention, notamment les sites de clivage par protéase tel que le site de clivage de la protéase TEV (protéase du virus de la gravure du tabac).

Dans de tels modes de mise en œuvre, le procédé selon l'invention comprend de préférence une étape de clivage de la protéine de fusion, par une enzyme adéquate, au niveau de ce site de clivage enzymatique, de sorte à séparer la protéine d'intérêt de l'étiquette protéique.

Une telle étape de clivage de la protéine de fusion n'est cependant qu'optionnelle, et n'est en particulier nécessaire que lorsque la fusion de l'étiquette protéique à la protéine d'intérêt modifie l'activité de cette dernière utile pour l'application visée de la protéine d'intérêt. Lorsque la fusion de l'étiquette protéique à la protéine d'intérêt ne modifie pas l'activité de cette dernière, et n'induit pas non plus d'effet secondaire néfaste dans le cadre de l'application visée, alors une étape de séparation de la protéine d'intérêt et de l'étiquette protéique n'est nullement nécessaire. En particulier, les protéines Mmi1 du genre *Schizosaccharomyces* interagissent avec l'ARN de manière tout à fait différente des protéines à domaine YTH des mammifères, si bien que la protéine de fusion préparée et purifiée selon l'invention peut avantageusement être administrée, telle quelle, à un mammifère, en particulier à un humain, sans que l'étiquette protéique ne vienne y perturber les processus biologiques normaux.

De manière tout à fait avantageuse, la présence de l'étiquette protéique selon l'invention en fusion avec la protéine d'intérêt n'entraine aucun effet indésirable dans les cellules d'eucaryotes supérieurs. Cette étiquette protéique ne présente aucune toxicité pour ces cellules, et n'y provoque pas de modification de la localisation de la protéine d'intérêt.

L'étape de préparation de la protéine de fusion peut être réalisée de toute manière conventionnelle, en particulier par voie biologique, en mettant en œuvre des techniques de génie génétique classiques en elles-mêmes.

En particulier, la protéine de fusion peut être préparée par transfection d'un organisme hôte adéquat avec une molécule d'acide nucléique codant pour la protéine de fusion, ou transformation d'un organisme hôte adéquat avec un vecteur d'expression dans lequel le gène hybride codant pour la protéine de fusion est lié de manière opérante à une séquence d'ADN contrôlant son expression ; et culture de cet organisme hôte dans des conditions permettant l'expression de la protéine de fusion, de telles conditions étant classiques en elles-mêmes et bien connues de l'homme du métier.

Les organismes hôtes pouvant être utilisés à cet effet incluent, de manière non limitative, les bactéries gram-positives et gram-négatives telles que des souches *d'Escherichia coli* ou *Bacillus subtilis,* des levures telles que des souches de *Saccharomyces cerevisiae,* et des organismes eucaryotes supérieures, notamment des lignées cellulaires mammifères.

Le gène hybride codant pour la protéine de fusion peut être préparé par des méthodes de recombinaison d'ADN classiques ou par des méthodes de synthèse génique également classiques en elles-mêmes. Il peut être incorporé dans tout vecteur d'expression de protéine classique.

A l'issue de cette étape de préparation du procédé selon l'invention, la protéine de fusion produite est séparée de l'organisme hôte et du milieu de culture par mise en présence du milieu la contenant, le cas échéant après une lyse cellulaire (une telle étape de lyse cellulaire n'étant pas nécessaire lorsque la protéine de fusion à séparer du milieu est du type à adressage extracellulaire) la molécule d'ARN cible, greffée sur un support solide ou couplée de manière covalente à un ligand de capture.

Dans des modes de mise en œuvre particuliers de l'invention, cette molécule d'acide ribonucléique contient une ou plusieurs répétitions du motif de séquence UNAAAC. Ces différentes répétitions peuvent aussi bien être contigües, qu'espacées les unes des autres par une séquence espaceur.

La molécule d'acide ribonucléique contenant au moins un motif de séquence nucléotidique UNAAAC peut comprendre, au sein de ce motif et/ou en tout autre position :
- au moins un nucléotide chimiquement modifié, en particulier un nucléotide 2'-O-méthylé, une telle modification étant réalisée de manière classique en elle-même, notamment au niveau du motif ribose ;
- et/ou au moins un acide nucléique bloqué, dit LNA (pour l'anglais « locked nucleic acid »), c'est-à-dire un analogue d'acide nucléique contenant un pont méthylène entre l'hydroxyle en position 2 et l'atome en position 4 du sucre ;
- et/ou au moins une liaison internucléotidique phosphorothiate.

L'étape de mise en présence de la protéine de fusion avec la molécule d'acide ribonucléique contenant au moins un motif de séquence nucléotidique UNAAAC est de préférence réalisée pendant une durée comprise entre 5 minutes et 1 heure, de préférence comprise entre 10 minutes et 30 minutes.

Le support solide sur lequel est greffé le partenaire d'affinité du ligand de capture ou la molécule d'acide ribonucléique peut être de tout type classique en lui-même, notamment pour la réalisation de procédés de séparation par immunoprécipitation. Il peut par exemple consister en des billes d'agarose ou de polystyrène, par exemple à propriétés magnétiques, pouvant notamment être isolées du milieu les contenant par des aimants.

Dans des modes de mise en œuvre particuliers de l'invention, le support solide est un support de chromatographie, classique en lui-même, par exemple un polymère réticulé à base de polysaccharide, tel que le dextrane ou l'agarose, ou de polyacrylamide, notamment sous forme de billes poreuses, ou de polystyrène.

Pour la mise en œuvre du procédé selon l'invention, le support de chromatographie est alors de préférence contenu dans une colonne (ou tube), dans laquelle est introduit le milieu contenant la protéine de fusion, le cas échéant liée à la molécule d'acide ribonucléique lorsque le procédé selon l'invention met en œuvre un ligand de capture couplé à cette dernière, pour la fixation de la protéine de fusion au support de chromatographie selon le principe de la chromatographie d'affinité, cette affinité étant soit entre le ligand de capture et son partenaire d'affinité greffé sur le support, soit entre la protéine de fusion, plus précisément l'étiquette protéique, et la molécule d'acide ribonucléique greffée sur le support.

Une telle colonne de chromatographie peut être opérée en mode par lots ou en continu.

Préférentiellement, préalablement à sa mise en présence avec la protéine de fusion, le support de chromatographie est équilibré, de manière classique en lui-même et selon les préconisations du fabricant, notamment par un tampon d'équilibration aqueux. Ce tampon d'équilibration peut contenir un agent dénaturant ou un détergent tel que le chlorure de guanidinium, l'urée ou le Triton^{®} X-100.

La séparation de la protéine de fusion et du support solide peut être réalisée de différentes manières, comprenant toutes une étape d'élution de la protéine d'intérêt (seule ou sous forme de protéine de fusion avec l'étiquette protéique). Cette élution peut être réalisée à pH constant ou avec des gradients de pH décroissant linéairement ou de manière discontinue. Les conditions d'élution optimales sont aisément déterminables par l'homme du métier, par des expériences de routine, pour chaque protéine d'intérêt donnée.

Le tampon d'élution peut contenir un agent dénaturant ou un détergent tel que le chlorure de guanidinium, l'urée ou le Triton^{®} X-100. L'addition d'un tel agent dénaturant ou détergent facilite la purification, même lorsque la protéine d'intérêt est peu soluble en solution aqueuse.

Dans les modes de mise en œuvre particuliers de l'invention dans lesquels un site de clivage enzymatique est inséré entre la protéine d'intérêt et l'étiquette protéique, la séparation de la protéine d'intérêt et du support solide peut être réalisée par clivage au niveau du site de clivage enzymatique. La protéine d'intérêt peut alors être récupérée par élution, comme indiqué ci-dessus.

Dans des variantes de l'invention, la protéine de fusion peut être séparée du support solide par élution chimique, notamment au moyen d'une solution à base de glycine de faible pH, puis la protéine d'intérêt peut optionnellement être dissociée de l'étiquette protéique par clivage au niveau du site de clivage enzymatique qui les sépare.

Dans des modes de mise en œuvre particuliers de l'invention, un site de clivage est inséré entre la molécule d'acide ribonucléique contenant au moins un motif de séquence nucléotidique UNAAAC et le support solide ou le ligand de capture. Ce site de clivage peut être du type clivable par une enzyme RNAse spécifique. Dans une telle configuration, la séparation de la protéine d'intérêt et du support de chromatographie peut être réalisée par clivage au niveau de ce site de clivage. La protéine de fusion peut alors être récupérée par élution, comme indiqué ci-dessus.

La séparation de la protéine de fusion et du support solide peut autrement être réalisée par clivage chimique au sein de la molécule d'ARN immobilisée, directement ou indirectement, par l'intermédiaire du couple « ligand de capture / partenaire d'affinité », sur le support solide.

Dans des modes de mise en œuvre particuliers de l'invention, l'étiquette protéique contient, en plus du fragment de la protéine Mmi1 en contenant les 173 ou les 174 acides aminés C-terminaux, au moins un domaine choisi parmi les domaines de Mmi1 de *Schizosaccharomyces* de séquence d'acides aminés suivants :
- RSVWXaa₁Xaa₂Xaa₃Xaa₄Xaa₅Xaa₆P (SEQ ID No : 13) (domaine 2 de Mmi1 de *Schizosaccharomyces),* où Xaa₁ représente un résidu thréonine, sérine ou alanine, Xaa₂ représente un résidu thréonine, arginine, lysine ou sérine, Xaa₃ représente un résidu histidine ou arginine, Xaa₄ représente un résidu thréonine ou proline, Xaa₅ représente un résidu glycine, alanine ou arginine et Xaa₆ représente un résidu acide glutamique ou acide aspartique, ce domaine étant désigné dans la présente description comme le « domaine 2 » ;
- FXaa₇SPLKRXaa₈APXaa₉SXaa₁₀Xaa₁₁Xaa₁₂Xaa₁₃Xaa₁₄Xaa₁₅R (SEQ ID No : 14) (domaine 3 de Mmi1 de *Schizosaccharomyces*), où Xaa₇ représente un résidu sérine ou thréonine, Xaa₈ représente un résidu proline ou glycine, Xaa₉ représente un résidu acide glutamique ou acide aspartique, Xaa₁₀ représente un résidu histidine, arginine ou lysine, Xaa₁₁ représente un résidu acide aspartique ou acide glutamique, Xaa₁₂ représente un résidu alanine ou tyrosine, Xaa₁₃ est nul ou représente un résidu proline, Xaa₁₄ représente un résidu isoleucine ou méthionine et Xaa₁₅ représente un résidu glycine ou acide aspartique, ce domaine étant désigné dans la présente description comme le « domaine 3 » ;
- YDFXaa₁₆RHCTDYGHSYXaa₁₇WPYFRSXaa₁₈RREXaa₁₉Xaa₂₀Xaa₂₁Y (SEQ ID No : 15) (domaine 4 de Mmi1 de *Schizosaccharomyces),* où Xaa₁₆ représente un résidu sérine, thréonine ou tyrosine, Xaa₁₇ représente un résidu acide glutamique ou acide aspartique, Xaa₁₈ représente un résidu leucine ou valine, Xaa₁₉ est nul ou représente un résidu sérine, Xaa₂₀ représente un résidu leucine ou méthionine et Xaa₂₁ représente un résidu arginine, leucine ou méthionine, ce domaine étant désigné dans la présente description comme le « domaine 4 » ;
- QPPXaa₂₂KRRTLXaa₂₃Xaa₂₄P (SEQ ID No : 16) (domaine 5 de Mmi1 de *Schizosaccharomyces),* où Xaa₂₂ représente un résidu proline, sérine ou leucine, Xaa₂₃ représente un résidu sérine ou leucine et Xaa₂₄ représente un résidu proline ou sérine, ce domaine étant désigné dans la présente description comme le « domaine 5 » ;
- Xaa₂₅AXaa₂₆Xaa₂₇SPXaa₂₈Xaa₂₉Xaa₃₀Xaa₃₁PXaa₃₂Xaa₃₃H (SEQ ID No : 17) (domaine 6 de Mmi1 de *Schizosaccharomyces),* où Xaa₂₅ représente un résidu arginine ou acide aspartique, Xaa₂₆ représente un résidu sérine ou glycine, Xaa₂₇ représente un résidu histidine ou acide aspartique, Xaa₂₈ représente un résidu sérine, glycine ou leucine, Xaa₂₉ représente un résidu leucine ou phénylalanine, Xaa₃₀ représente un résidu leucine, isoleucine ou sérine, Xaa₃₁ représente un résidu acide glutamique ou acide aspartique, Xaa₃₂ représente un résidu tyrosine ou thréonine et Xaa₃₃ représente un résidu alanine ou thréonine, ce domaine étant désigné dans la présente description comme le « domaine 6 » ;
- RXaa₃₄EKPKXaa₃₅RAXaa₃₆TPPP (SEQ ID No : 18) (domaine 7 de Mmi1 de *Schizosaccharomyces),* où Xaa₃₄ représente un résidu lysine ou arginine, Xaa₃₅ représente un résidu alanine, proline ou thréonine et Xaa₃₆ représente un résidu sérine ou proline, ce domaine étant désigné dans la présente description comme le « domaine 7 ».

L'étiquette protéique peut contenir deux ou plus des domaines 2 à 7 ci-dessus, y compris la totalité de ces domaines, toute combinaison de deux ou plus de ces domaines entrant dans le cadre de l'invention.

Préférentiellement, dans la séquence d'acides aminés de chacun des domaines 2 à 7 ci-dessus, les acides aminés variables sont choisis pour que la séquence d'acides aminés de chacun des domaines corresponde à la séquence d'acides aminés d'un domaine de la protéine Mmi1 d'un microorganisme du genre *Schizosaccharomyces,* préférentiellement le même que celui dont est issu ledit fragment contenant le domaine YTH+ de la protéine. Les différents domaines, ainsi que le domaine YTH+, contenus dans l'étiquette protéique selon l'invention sont alors de préférence positionnés les uns par rapport aux autres selon leur positionnement normal dans la protéine native, ces domaines étant alors contigus ou séparés par les séquences intercalaires natives de la protéine, ou par des séquences obtenues par substitution, addition ou délétion par rapport à ces séquences intercalaires natives.

Dans des modes de mise en œuvre particuliers de l'invention, l'étiquette protéique comprend le domaine YTH+ de la protéine Mmi1 de *Schizosaccharomyces pombe* et au moins un domaine de cette protéine choisi parmi les domaines suivants, ou plusieurs de ces domaines, voire la totalité, selon toutes les combinaisons envisageables :
- domaine 2 : s'étendant de l'acide aminé en position 40 (arginine) à l'acide aminé en position 50 (proline) de la protéine Mmi1, de séquence : RSVWTTHTGEP (SEQ ID No : 19), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 3 : s'étendant de l'acide aminé en position 64 (phénylalanine) à l'acide aminé en position 82 (arginine) de la protéine Mmi1, de séquence : FSSPLKRPAPESHDAPIGR (SEQ ID No : 20), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 4 : s'étendant de l'acide aminé en position 97 (tyrosine) à l'acide aminé en position 125 (tyrosine) de la protéine Mmi1, de séquence : YDFSRHCTDYGHSYEWPYFRSLRRESMLY (SEQ ID No : 21), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 5 : s'étendant de l'acide aminé en position 169 (glutamine) à l'acide aminé en position 180 (proline) de la protéine Mmi1, de séquence : QPPPKRRTLSPP (SEQ ID No : 22), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 6 : s'étendant de l'acide aminé en position 258 (arginine) à l'acide aminé en position 272 (histidine) de la protéine Mmi1, de séquence : RASHSPSLLEPYAH (SEQ ID No : 23), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 7 : s'étendant de l'acide aminé en position 302 (arginine) à l'acide aminé en position 315 (proline) de la protéine Mmi1, de séquence : RKEKPKARASTPPP (SEQ ID No : 24), ou une région protéique de séquence d'acides aminés présentant au moins 90 %, de préférence au moins 95%, de préférence encore au moins 98 %, et préférentiellement au moins 99 %, d'identité avec cette séquence d'acides aminés.

Comme indiqué ci-avant, ces différents domaines, ainsi que le domaine YTH+, contenus dans l'étiquette protéique, sont de préférence positionnés les uns par rapport aux autres selon leur positionnement normal dans la protéine native, ces domaines étant alors contigus ou séparés par les séquences intercalaires natives de la protéine, ou par des séquences obtenues par substitution, addition ou délétion par rapport à ces séquences intercalaires natives.

Dans des modes de mise en œuvre particuliers de l'invention, l'étiquette protéique comprend, ou consiste en, la protéine Mmi1 de *Schizosaccharomyces pombe* délétée des 30 acides aminés en position N-terminale. L'étiquette protéique comprend alors, ou consiste en, la séquence d'acides aminés de séquence SEQ ID No : 25.

Dans des modes de mise en œuvre particuliers de l'invention, l'étiquette protéique comprend le domaine YTH+ de la protéine Mmi1 de *Schizosaccharomyces japonicus* et au moins un domaine de cette protéine choisi parmi les domaines suivants, ou plusieurs de ces domaines, voire la totalité, selon toutes les combinaisons envisageables :
- domaine 2 : s'étendant de l'acide aminé en position 38 (arginine) à l'acide aminé en position 48 (proline) de la protéine Mmi1, de séquence : RSVWAKHPNDP (SEQ ID No : 26), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 3 : s'étendant de l'acide aminé en position 61 (phénylalanine) à l'acide aminé en position 78 (arginine) de la protéine Mmi1, de séquence : FSSPLKRGAPDSKEYMDR (SEQ ID No : 27), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 4 : s'étendant de l'acide aminé en position 93 (tyrosine) à l'acide aminé en position 120 (tyrosine) de la protéine Mmi1, de séquence : YDFYRHCTDYGHSYDWPYFRSLRRELAY (SEQ ID No : 28), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 5 : s'étendant de l'acide aminé en position 166 (glutamine) à l'acide aminé en position 177 (proline) de la protéine Mmi1, de séquence : QPPLKRRTLLSP (SEQ ID No : 29), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 6 : s'étendant de l'acide aminé en position 245 (acide aspartique) à l'acide aminé en position 258 (histidine) de la protéine Mmi1, de séquence : DAGDSPLFSEPTAH (SEQ ID No : 30), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 7 : s'étendant de l'acide aminé en position 292 (arginine) à l'acide aminé en position 305 (proline) de la protéine Mmi1, de séquence : RREKPKTRAPTPPP (SEQ ID No : 31), ou une région protéique de séquence d'acides aminés présentant au moins 90 %, de préférence au moins 95%, de préférence encore au moins 98 %, et préférentiellement au moins 99 %, d'identité avec cette séquence d'acides aminés.

Dans des modes de mise en œuvre alternatifs de l'invention, l'étiquette protéique comprend le domaine YTH+ de la protéine Mmi1 de *Schizosaccharomyces octosporus* et au moins un domaine de cette protéine choisi parmi les domaines suivants, ou plusieurs de ces domaines, voire la totalité, selon toutes les combinaisons envisageables :
- domaine 2 : s'étendant de l'acide aminé en position 39 (arginine) à l'acide aminé en position 49 (proline) de la protéine Mmi1, de séquence : RSVWSNRPAEP (SEQ ID No : 32), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 3 : s'étendant de l'acide aminé en position 61 (phénylalanine) à l'acide aminé en position 79 (arginine) de la protéine Mmi1, de séquence : FTSPLKRPAPDSREAPMGR (SEQ ID No : 33), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 4 : s'étendant de l'acide aminé en position 94 (tyrosine) à l'acide aminé en position 122 (tyrosine) de la protéine Mmi1, de séquence : YDFTRHCTDYGHSYEWPYFRSVRRESLMY (SEQ ID No : 34), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 5 : s'étendant de l'acide aminé en position 170 (glutamine) à l'acide aminé en position 181 (proline) de la protéine Mmi1, de séquence : QPPSKRRTLSPP (SEQ ID No : 35) ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 6 : s'étendant de l'acide aminé en position 253 (arginine) à l'acide aminé en position 266 (histidine) de la protéine Mmi1, de séquence : RASHSPGLIDPYTH (SEQ ID No : 36), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 7 : s'étendant de l'acide aminé en position 293 (arginine) à l'acide aminé en position 306 (proline) de la protéine Mmi1, de séquence : RKEKPKPRAPTPPP (SEQ ID No : 37), ou une région protéique de séquence d'acides aminés présentant au moins 90 %, de préférence au moins 95%, de préférence encore au moins 98 %, et préférentiellement au moins 99 %, d'identité avec cette séquence d'acides aminés.

Dans des modes de mise en œuvre encore alternatifs de l'invention, l'étiquette protéique comprend le domaine YTH+ de la protéine Mmi1 de *Schizosaccharomyces cryophilus* et au moins un domaine de cette protéine choisi parmi les domaines suivants, ou plusieurs de ces domaines, voire la totalité, selon toutes les combinaisons envisageables :
- domaine 2 : s'étendant de l'acide aminé en position 39 (arginine) à l'acide aminé en position 49 (proline) de la protéine Mmi1, de séquence : RSVWSSRPAEP (SEQ ID No : 38), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 3 : s'étendant de l'acide aminé en position 61 (phénylalanine) à l'acide aminé en position 79 (proline) de la protéine Mmi1, de séquence : FTSPLKRPAPDSREAPIGR (SEQ ID No : 39), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 4 : s'étendant de l'acide aminé en position 94 (tyrosine) à l'acide aminé en position 122 (tyrosine) de la protéine Mmi1, de séquence : YDFTRHCTDYGHSYEWPYFRSVRRESLMY (SEQ ID No : 40), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 5 : s'étendant de l'acide aminé en position 169 (glutamine) à l'acide aminé en position 180 (proline) de la protéine Mmi1, de séquence : QPPSKRRTLSPP (SEQ ID No : 41) ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 6 : s'étendant de l'acide aminé en position 252 (acide aspartique) à l'acide aminé en position 265 (histidine) de la protéine Mmi1, de séquence : RASHSPSLIDPYAH (SEQ ID No : 42), ou une région protéique de séquence d'acides aminés présentant au moins 90 %, de préférence au moins 95%, de préférence encore au moins 98 %, et préférentiellement au moins 99 %, d'identité avec cette séquence d'acides aminés ;
- domaine 7 : s'étendant de l'acide aminé en position 292 (arginine) à l'acide aminé en position 305 (proline) de la protéine Mmi1, de séquence : RKEKPKPRAPTPPP (SEQ ID No : 43), ou une région protéique de séquence d'acides aminés présentant au moins 90 %, de préférence au moins 95%, de préférence encore au moins 98 %, et préférentiellement au moins 99 %, d'identité avec cette séquence d'acides aminés.

La protéine de fusion selon l'invention peut en outre comprendre d'autres étiquettes chimiques ou protéiques, classiques en elles-mêmes, une ou plusieurs séquences de localisation subcellulaire, etc.

Une protéine de fusion recombinante, susceptible d'être obtenue à l'issue de l'étape de préparation d'une protéine de fusion du procédé de purification selon l'invention, ou à l'issue du procédé selon l'invention, sous forme purifiée, comprend une protéine d'intérêt fusionnée à une étiquette protéique, cette étiquette protéique comprenant au moins, ou étant constituée de : la protéine Mmi1 d'un microorganisme du genre *Schizosaccharomyces,* un fragment de cette protéine Mmi1 en comprenant au moins les 173 acides aminés C-terminaux, ou une protéine de séquence d'acides aminés ayant au moins 90 % d'identité, de préférence au moins 95 %, préférentiellement au moins 98 %, et encore préférentiellement au moins 99 % d'identité avec la séquence d'acides aminés de ladite protéine Mmi1 ou dudit fragment et capable de se lier à un motif d'acide ribonucléique de séquence nucléotidique UNAAAC.

Cette protéine de fusion peut présenter toute caractéristique ou combinaison de caractéristiques décrites ci-avant en référence au procédé de purification selon l'invention, et se rapportant à la protéine de fusion préparée / mise en œuvre. En particulier, un site de clivage enzymatique y est inséré entre la protéine d'intérêt et l'étiquette protéique.

Une molécule d'acide nucléique codant pour une telle protéine de fusion, particulièrement adaptée pour une mise en œuvre dans l'étape de préparation de la protéine de fusion d'un procédé de purification selon l'invention, peut en particulier être obtenue par toute méthode de génie génétique classique en elle-même.

Une telle molécule d'acide nucléique peut par exemple comprendre, dans le cadre de lecture, une séquence choisie parmi les séquences SEQ ID No : 2 et SEQ ID No : 44, correspondant aux séquences codant, respectivement, pour la protéine Mmi1 de *Schizosaccharomyces pombe,* de séquence d'acides aminés SEQ ID No : 1, et pour le domaine YTH+ de cette dernière, de séquence d'acides aminés SEQ ID No : 9.

Un vecteur d'expression comprenant une telle molécule d'acide nucléique, particulièrement adapté pour une mise en œuvre dans l'étape de préparation de la protéine de fusion d'un procédé de purification selon l'invention, peut être de tout type connu en lui-même pour une mise en œuvre en ingénierie génétique, notamment un plasmide, un cosmide, un virus, un bactériophage, contenant les éléments nécessaires pour la transcription et la traduction de la séquence codant pour la protéine de fusion selon l'invention.

Il comprend notamment les éléments suivants, liés de manière fonctionnelle : un promoteur situé en 5' d'une séquence nucléotidique codant pour la protéine de fusion selon l'invention, et des signaux de terminaison de la transcription en 3' de cette séquence.

Une cellule hôte comprenant une telle protéine de fusion, une telle molécule d'acide nucléique et/ou un tel vecteur d'expression, particulièrement adaptée pour une mise en œuvre dans l'étape de préparation de la protéine de fusion d'un procédé de purification selon l'invention, peut aussi bien être une cellule procaryote, en particulier bactérienne, notamment pour la production en masse de la protéine de fusion selon l'invention, qu'une cellule eucaryote, cet eucaryote pouvant être inférieur ou supérieur, par exemple une cellule de levures, d'invertébrés ou de mammifères. En particulier, entrent dans le cadre de l'invention des lignées cellulaires exprimant, de manière stable, inductible ou constitutive, ou bien transitoire, une protéine de fusion selon l'invention.

La protéine de fusion mise en œuvre selon l'invention peut être préparée par toute méthode conventionnelle connue de l'homme du métier. Elle peut notamment être obtenue par génie génétique ou par synthèse chimique.

Un procédé de préparation d'une telle protéine de fusion, pouvant être mis en œuvre pour réaliser l'étape de préparation de la protéine de fusion du procédé de purification selon l'invention, comprend la transfection d'une cellule hôte avec une molécule d'acide nucléique telle que définie ci-dessus ou la transformation d'une cellule hôte avec un vecteur d'expression tel que défini ci-dessus ; et la culture de cette cellule hôte dans des conditions permettant l'expression de la protéine de fusion visée.

Un autre objet de l'invention est une molécule d'acide ribonucléique contenant au moins un motif de séquence nucléotidique UNAAAC, couplée à un ligand de capture, par exemple à une molécule de biotine.

Cette molécule d'acide ribonucléique peut présenter toute caractéristique ou combinaison de caractéristiques décrites ci-avant en référence au procédé de purification selon l'invention, et se rapportant à la molécule d'acide ribonucléique mise en œuvre.

Un autre aspect de l'invention est un support solide pour la mise en œuvre d'un procédé de purification d'une protéine d'intérêt selon l'invention. Une molécule d'acide ribonucléique contenant au moins un motif de séquence nucléotidique UNAAAC est greffée sur ce support solide.

Ce support solide peut présenter toute caractéristique ou combinaison de caractéristiques décrites ci-avant en référence au procédé de purification selon l'invention, et se rapportant au support solide greffé mis en œuvre.

Selon un autre aspect, l'invention concerne un kit pour la mise en œuvre d'un procédé de purification d'une protéine d'intérêt selon l'invention. Ce kit contient une molécule d'acide ribonucléique contenant au moins un motif de séquence nucléotidique UNAAAC greffée sur un support solide ou couplée à un ligand de capture, et au moins un des constituant suivants, par exemple ces deux constituants :
- un vecteur d'expression comprenant, sous le contrôle d'un promoteur, une molécule d'acide nucléique codant pour une étiquette protéique comprenant au moins, ou constituée de : la protéine Mmi1 d'un microorganisme du genre *Schizosaccharomyces,* un fragment de cette protéine Mmi1 en comprenant au moins les 173 acides aminés C-terminaux, ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC ; et un site permettant l'insertion, en 5' ou en 3' par rapport à ladite molécule d'acide nucléique codant pour ladite étiquette protéique, d'une molécule d'acide nucléique codant pour ladite protéine d'intérêt de sorte à permettre l'expression d'une protéine de fusion contenant ladite protéine d'intérêt et ladite étiquette protéique ;
- et/ou des instructions pour la mise en œuvre des étapes d'un procédé selon l'invention.

Ce kit peut en outre contenir, dans le cas dans lequel la molécule d'acide ribonucléique est couplée à un ligand de capture, un support solide sur lequel est greffé un partenaire d'affinité de ce ligand de capture.

Chacun de ces constituants peut répondre à l'une ou plusieurs des caractéristiques décrites ci-avant en référence au procédé de purification selon l'invention, et se rapportant à ce constituant.

Le vecteur d'expression, permettant le clonage de la molécule d'acide nucléique codant pour la protéine d'intérêt de sorte à former une molécule d'acide nucléique chimère codant pour la protéine de fusion, et l'expression de cette dernière, peut être de tout type connu en lui-même pour une mise en œuvre en ingénierie génétique, notamment un plasmide, un cosmide, un virus, un bactériophage, etc. Il contient les éléments nécessaires pour le clonage dans un site d'insertion adéquat d'une molécule d'acide nucléique codant pour la protéine d'intérêt, ainsi que la transcription et la traduction de la séquence chimérique codant pour cette protéine de fusion « protéine d'intérêt - étiquette protéique » (ou « étiquette protéique - protéine d'intérêt ») ainsi obtenue. Il comprend en particulier les éléments suivants, liés de manière fonctionnelle : un promoteur situé en 5' d'une molécule d'acide nucléique codant pour l'étiquette protéique selon l'invention, un site d'insertion d'une molécule d'acide nucléique codant pour la protéine d'intérêt dans le même cadre de lecture que la molécule d'acide nucléique codant pour l'étiquette protéique selon l'invention, et des signaux de terminaison de la transcription en 3' de ces éléments.

Le kit peut également contenir une cellule hôte apte à être transformée par un vecteur d'expression comprenant, sous le contrôle d'un promoteur : une molécule d'acide nucléique codant pour une étiquette protéique comprenant au moins, ou constituée de la protéine Mmi1 d'un microorganisme du genre *Schizosaccharomyces,* un fragment de cette protéine Mmi1 en comprenant au moins les 173 acides aminés C-terminaux, ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC ; et un site permettant l'insertion, en 5' ou en 3' par rapport à cette molécule d'acide nucléique codant pour cette étiquette protéique, d'une molécule d'acide nucléique codant pour la protéine d'intérêt, ce site étant configuré pour permettre l'expression d'une protéine de fusion contenant la protéine d'intérêt et l'étiquette protéique.

Cette cellule hôte peut aussi bien être une cellule procaryote, en particulier bactérienne, notamment pour la production en masse de la protéine de fusion selon l'invention, qu'une cellule eucaryote, cet eucaryote pouvant être inférieur ou supérieur, par exemple une cellule de levure, d'invertébrés ou de mammifères.

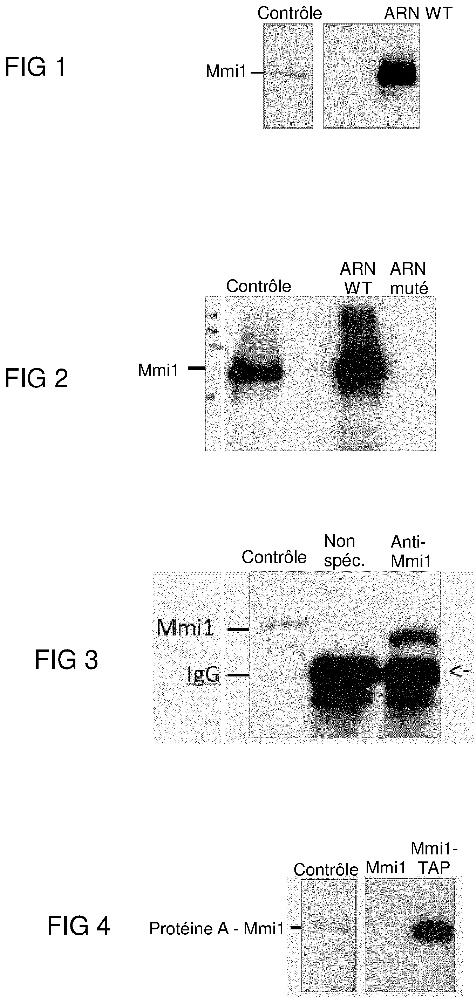

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 6, dans lesquelles :
La figure 1 montre une photographie d'une membrane de western blot, la révélation ayant été réalisée au moyen d'un anticorps anti-Mmi1, obtenue après expression de la protéine Mmi1 de *S. pombe* chez *E. coli,* lyse cellulaire, mise en présence (piste de droite, « ARN WT ») ou non (piste de gauche, « Contrôle ») pendant 15 min du lysat dilué au 1/100^{ème} avec une molécule d'ARN « ARN WT » contenant un motif UNAAAC (conforme à l'invention), récupération des protéines liées à la molécule d'ARN, et séparation des protéines par électrophorèse SDS.
La figure 2 montre une photographie d'une membrane de western blot, la révélation ayant été réalisée au moyen d'un anticorps anti-Mmi1, obtenue après expression de la protéine Mmi1 de *S. pombe* chez *E. coli,* lyse cellulaire, mise en présence du lysat dilué au 1/10^{ème} avec une molécule d'ARN « ARN WT » contenant un motif UNAAAC (conforme à l'invention, piste du milieu) ou une molécule d'ARN « ARN muté » contenant un motif CNAAAC (non conforme à l'invention - témoin négatif, piste de droite) ou absence de telle mise en présence (piste de gauche, « Contrôle »), récupération des protéines liées à la molécule d'ARN, et séparation des protéines par électrophorèse SDS-PAGE.
La figure 3 montre une photographie d'une membrane de western blot, la révélation ayant été réalisée au moyen d'un anticorps anti-Mmi1, obtenue après expression de la protéine Mmi1 de *S. pombe* chez *S. pombe,* lyse cellulaire, mise en présence (ou absence de mise en présence : piste de gauche, « Contrôle ») pendant 1 h du lysat dilué au 1/10^{ème} avec un anticorps anti-Mmi1 (piste de droite) ou un anticorps non dirigé contre Mmi1 (IgG humain, piste du milieu, « Non spec. »), récupération des protéines liées à l'anticorps, et séparation des protéines par électrophorèse SDS-PAGE.
La figure 4 montre une photographie d'une membrane de western blot, la révélation ayant été réalisée au moyen d'un anticorps anti-Mmi1, obtenue après expression de la protéine Mmi1 de *S. pombe* en fusion avec la protéine A (piste de droite, « Mmi1-TAP ») ou seule (piste du milieu, « Mmi1 ») chez *S. pombe,* lyse cellulaire, mise en présence (ou absence de mise en présence : piste de gauche, « Contrôle ») pendant 1 h du lysat dilué au 1/10^{ème} avec des IgG greffés sur billes, récupération des protéines liées aux IgG, et séparation des protéines par électrophorèse SDS-PAGE.

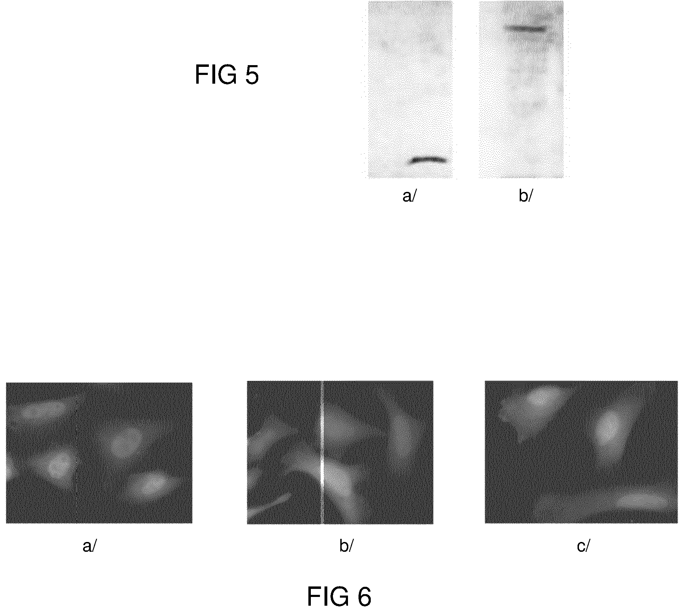

La figure 5 montre des photographies de membranes de western blot, la révélation ayant été réalisée au moyen d'un anticorps anti-GFP, obtenues après expression dans des cellules HEK293, respectivement, en a/ de la GFP et en b/ de la protéine Mmi1 fusionnée à la GFP.
La figure 6 montre des images, acquises par microscopie à fluorescence, de cellules HEK293 surexprimant, en a/ la protéine de fusion de la GFP et de la protéine Mmi1, la GFP étant du côté C-terminal, en b/ la GFP seule, et en c/ la protéine de fusion de la GFP et de la protéine Mmi1, la GFP étant du côté N-terminal.

### A/ Matériel et méthodes

A.1/ Cellules et plasmides utilisés pour la production des protéines
- Bactéries : souche *Escherichia coli* BL21 et plasmide pETM11 (les séquences d'ADN codant pour la protéine Mmi1 ont été clonées dans ce plasmide en utilisant les sites de restriction *Nco*I et *Eco*RI) ;
- Levures : *Schizosaccharomyces Pombe ;*
- Cellules humaines : lignées HeLa et HEK293, et plasmides pEGFP-C3 et pEGFP-N3 (Clontech) (les séquences d'ADN codant pour la protéine Mmi1 ont été clonées dans ces plasmides en utilisant les sites de restriction *Xhol* et *Bam*HI).

Dans toutes les expériences, la protéine Mmi1 est celle de *S. pombe* (protéine Mmi1 complète, de séquence d'acides aminés SEQ ID No : 1).

### A.2/ Expression bactérienne

J1 : transformer les cellules de la souche BL21 avec le plasmide pETM11 contenant Mmi1 en suivant la procédure standard utilisée pour les cellules Top10 (en appliquant le protocole conventionnel de transformation bactérienne) ; J2 : prélever une colonie et l'inoculer dans 50 ml de milieu LB additionné de kanamycine à 50 mM et laisser la culture pousser à 37 °C sous agitation (180 tr/min) ; J3 : transférer l'inoculum dans 200ml de milieu LB additionné de kanamycine à 50 mM et le mettre de nouveau sous agitation à 37 °C pendant 1 h ; vérifiez la densité optique des cellules - lorsqu'elle atteint 0,8, prendre un aliquote de cellules non induites (5 ml), afin de constituer le contrôle négatif d'induction (cellules non-induites) - faire descendre la température de l'incubateur à 18 °C et laisser la culture 15 min en agitation avant d'induire l'expression par ajout d'IPTG - ajouter 0,2 ml d'IPTG 1M dans une culture de 200 ml (concentration finale 1 mM) et laisser la culture sous agitation pendant la nuit ; J4 : centrifuger les cellules par fractions de 50 ml à 5000 g pendant 20 min à 4 °C, puis congeler les culots à -80 °C.

### A.3/ Liaison de Mmi1 à l'ARN chez E. coli

Lyse cellulaire : un culot de cellules *E. coli* BL21 exprimant la protéine Mmi1 est décongelé sur la glace. Les cellules sont ensuite lysées par sonication dans 5 ml de tampon de lyse (50 mM Tris pH 7,6, 150 mM KCI, 5mM MgCl2, 1 mM EDTA, 1% Triton X-100, 10% glycérol, 5 mM DTT, 1 mM PMSF, 1µg/ml de LABP (Cocktail d'inhibiteurs de protéases (Sigma) : Aprotinine réf. 10236624001, Bestatine réf. 10874515001, Leupeptine réf. 10874515001, Pepstatine réf. 11359053001). Le lysat est ensuite transféré dans un nouveau tube de 15ml et centrifugé 10 min à 14000 g à 4 °C, puis soniqué selon une alternance « 10 s sonication/10 s repos » à 85% de la puissance maximale (sonicateur Vibracell 75186, ThermoFisher) pour une durée totale de temps de sonication de 2 min. Préparation d'ARNs biotinylés_: pour chaque immunoprécipitation, 100 ng des ARNs nommés « ARN WT » et « ARN muté » sont dénaturés 2 min à 90 °C, puis les ARNs dénaturés sont incubés 20 min à température ambiante dans le tampon structurant (10 mM Tris pH7, 0,1 M KCI, 10mM MgCl₂) avant d'être incubés avec le lysat. Les ARNs utilisés ont été obtenus chez Eurogentec et présentent les séquences suivantes :
ARN WT : 5' Biot-GGAUCCUUAAACAGAUCU 3' (SEQ ID No : 45) (séquence artificielle, substrat pour liaison de Mmi1) - présentant un motif UNAAAC conformément à l'invention,
ARN muté : 5' Biot-GGAUCCCUAAACAGAUCU 3' (SEQ ID No : 46) (séquence artificielle, contrôle négatif pour liaison de Mmi1) - contrôle négatif ne présentant pas de motif UNAAAC mais un motif CNAAAC.
Liaison à l'ARN : il est réalisé une dilution au 1/100^{ème} ou au 1/10^{ème} de l'extrait cellulaire obtenu à l'issue de l'étape de lyse cellulaire. L'ARN biotinylé (100 ng) est ajouté à 100 µl d'extrait cellulaire, puis l'extrait est incubé sous agitation pendant 15 à 20 min à 4 °C. L'immunoprécipitation est réalisée par ajout de 15 µl de 10 mg/ml de Dynabeads^{®} M-280 Streptavidin (Invitrogen) et incubation pendant 30 min sous agitation. Les billes sont ensuite lavées trois fois avec 1 ml de tampon de lyse avec 5 min d'agitation à chaque lavage. L'élution de la protéine Mmi1 associée à l'ARN se fait par ébullition pendant 5 min dans le tampon SDS Laemmli 2X. L'efficacité de la liaison de la protéine à l'ARN est analysée par immunoblot (western blot), la séparation par électrophorèse SDS-PAGE étant effectuée sur gel de polyacrylamide 10% pendant 1 h à 180 V. Après transfert sur membrane de nitrocellulose, la détection de la protéine est réalisée au moyen d'un anticorps anti-Mmi1, tel que décrit dans la publication de Touat-Todeschini et al, EMBO J, 2017, 36:2626-2641. A cet effet, la membrane de nitrocellulose est incubée pendant une heure à température ambiante avec cet anticorps primaire anti-Mmi1 dilué au 1/1000 dans du TBS 0,1% tween (TBS-T) contenant 10%SVF, suivi de trois lavages de 5 min chacun avec du TBS-T. La membrane est ensuite incubée 45 min avec un anticorps secondaire couplé à l'HRP (DAKO, réf. P0448) dilué au 1/5000 dan du TBS-T contenant 1% de lait, puis lavée de nouveau 3 fois 5 min avec du TBS-T.

### A.4/ Expériences d'immunoprécipitation chez la levure

On utilise des cellules de *S. pombe* dans lesquelles Mmi1 a été surexprimée à l'aide d'un promoteur inductible (promoteur nmt1) inséré au locus endogène de Mmi1. Des cellules surexprimant Mmi1 en fusion avec l'étiquette TAP (pour l'anglais « Tandem Affinité Purification », purification d'affinité tandem, composée de la protéine A et de la CBP (protéine liant la calmoduline), sont également utilisées, dans lesquelles l'étiquette TAP a été rajoutée à la séquence endogène du gène mmi1 par l'approche conventionnelle de recombinaison homologue de produits de polymérisation par réaction en chaine chez la levure. Ces cellules sont cultivées à une densité optique (DO) de 1,2 dans un volume total de 50 ml de milieu de culture YEA.

Les étapes suivantes sont toutes réalisées à 4 °C.

Le tampon de lyse (LysBuff) (100 mM HEPES pH 7,5, 20 mM MgCl₂, 10% Glycérol, 10 mM EGTA, 0,1 M EDTA, 0,4% NP-40, 150 mM NaCl, 1 mM DTT, 1 mM PMSF, 1 µg/ml LABP) est utilisé pour la lyse et pour les lavages.

Lyse des cellules : reprendre les culots dans 400 µl de tampon de lyse LysBuff (2x) (mélanger le tube délicatement si nécessaire), puis ajouter des billes de verre et agiter 2x30 s dans un agitateur de billes, avec un temps de repos de 2 min dans la glace entre les deux cycles. Percer le fond des tubes avec une seringue 0,5 mm et placer les tubes de lysat dans des tubes de 5 ml à fond rond (refroidis dans la glace). Centrifuger à 4 °C pendant 1 min à 3000 tr/min, puis transférer les lysats dans de nouveaux tubes Eppendorf. Centrifuger 10 min à 13000 tr/min à 4 °C. La concentration de protéines dans chaque échantillon obtenu est estimée par la méthode de Bradford, de sorte à utiliser la même quantité de protéines totales dans chaque expérience d'immunoprécipitation.

Immunoprécipitation TAP : utiliser 15 µl d'anticorps IgG greffés sur des billes de résine de Sepharose^{®} (IgG Sepharose, Réf.17-0969-01, GE-Healthcare) par expérience d'immunoprécipitation. Réaliser 3 lavages des billes avec 500 µl de tampon de lyse LysBuff 2x. Mélanger les échantillons de protéines (la même quantité de protéines dans chaque échantillon) avec 15 µl de billes préparées dans l'étape précédente. Agiter délicatement à 4°C pendant 1 h.

Immunoprécipitation par anticorps anti-Mmi1 : les protéines (la même quantité dans chaque échantillon) sont mélangées à 2 µl d'anticorps anti-Mmi1 précité et le mélange est agité délicatement pendant 1 h à 4 °C. 20 µl de billes de résine de Sepharose^{®} sur lesquelles est greffée la protéine A (Sepharose^{®} Protéine A, Réf. 17-5280-01, GE-Healthcare) sont ajoutés au mélange, qui est ensuite agité de nouveau pendant 1 h à 4°C.

Lavages et élution : faire 3 lavages des billes avec 500 µl du LysBuff 2x en procédant de la même manière : lavage pendant 5 min avec agitation délicate puis centrifugation 2 min à 500 g. L'élution se fait par ébullition pendant 5 min dans le tampon SDS Laemmli 2X. Pour la détection par Western Blot, le même procédé que celui décrit plus haut en référence à l'expérience de liaison de Mmi1 à l'ARN chez *E. coli.* La détection du TAP se fait avec un anticorps anti-TAP.

### A.5/ Transfection des cellules humaines

La transfection est réalisée selon le protocole classique du fournisseur, en utilisant la Lipofectamine^{®} 3000 (ThermoFisher).

Les cellules sont ensuite soit lysées pour une détection par la méthode conventionnelle du western blot en utilisant un anticorps anti-GFP (Roche #11814460001), soit visualisées directement sur lame au microscope à fluorescence, selon des protocoles conventionnels.

La visualisation par western blot et par microscopie à fluorescence est réalisée après 48 h de transfection, selon des protocoles opératoires conventionnels.

### B/ Expérience 1

Cette expérience est réalisée avec la protéine Mmi1 produite chez *E. coli.*

Le gène de séquence SEQ ID No : 2 est cloné dans le plasmide, pour permettre l'expression par les cellules de la protéine Mmi1 de *S. pombe,* de séquence d'acides aminés SEQ ID No : 1 (protéine Mmi1 complète).

Schématiquement, un extrait obtenu par lyse cellulaire de cellules exprimant la protéine Mmi1 est mis en présence d'une ou l'autre des molécules d'ARN biotinylés en 5' « ARN WT » (conforme à l'invention) et « ARN muté » (non conforme à l'invention, témoin négatif), pendant une durée de 15 min, puis les molécules d'ARN (et les protéines fixées sur ces molécules) sont isolées du milieu au moyen de billes magnétiques Dynabead^{®} sur lesquelles est greffée la streptavidine, en utilisant la forte capacité de cette dernière à se lier à la biotine.

Les protéines fixées sur les billes sont éluées, et analysées par western blot, après séparation des protéines par électrophorèse sur gel SDS-PAGE, au moyen de l'anticorps anti-Mmi1.

Les résultats obtenus sont montrés sur la figure 1 pour une dilution au 1/100^{ème} de l'extrait de lyse cellulaire, pour l'ARN conforme à l'invention « ARN WT ». On observe clairement la présence de la protéine Mmi1 sur la membrane de western blot, et aucun bruit de fond, démontrant la spécificité de liaison du motif d'ARN UNAAAC avec la protéine Mmi1.

Les résultats obtenus sont montrés sur la figure 2 pour une dilution au 1/10^{ème} de l'extrait de lyse cellulaire, pour l'ARN conforme à l'invention « ARN WT » et pour l'ARN non conforme à l'invention « ARN muté ». La révélation par l'anticorps anti-Mmi1 révèle une bande de forte intensité correspondant au poids moléculaire de la protéine Mmi1 (54 kDa) lorsque l'ARN présente un motif UNAAAC conforme à l'invention (« ARN WT »). On n'observe aucune bande au poids moléculaire de la protéine Mmi1 lorsque la molécule d'ARN « ARN muté » a été mise en œuvre, ce qui témoigne de la spécificité de liaison de la protéine Mmi1 pour le motif UNAAAC.

Cette expérience démontre clairement que la protéine Mmi1 peut être purifiée avec un haut degré de pureté d'un milieu cellulaire complexe par interaction avec une molécule d'ARN comprenant un motif UNAAAC greffée sur un support solide.

### B/ Expérience 2 - comparaison avec une immunoprécipitation par anticorps

Cette expérience est réalisée avec la protéine Mmi1 endogène de *S. pombe,* de séquence d'acides aminés SEQ ID No : 1 (protéine Mmi1 complète).

Schématiquement, un extrait obtenu par lyse cellulaire de cellules exprimant la protéine Mmi1 est mis en présence pendant 1 h avec un anticorps anti-Mmi1.

Après séparation du milieu au moyen de billes de Sepharose^{®} sur lesquelles est greffée la protéine A, les protéines fixées sur les billes sont éluées, et analysées par western blot, après séparation des protéines par électrophorèse sur gel SDS-PAGE, au moyen de l'anticorps anti-Mmi1.

Les résultats obtenus sont montrés sur la figure 3. On observe bien, sur la piste associée aux protéines capturées par immunoprécipitation avec l'anticorps anti-Mmi1 (piste « anti-Mmi1 »), une bande correspondant au poids moléculaire de la protéine Mmi1, qui ne se retrouve pas sur les autres pistes. On observe cependant également une bande de très forte intensité correspondant à une contamination par les IgG présents dans l'extrait cellulaire initial (bande indiquée par une flèche à droite sur la figure).

En comparaison de cette purification par l'anticorps anti-Mmi1, le procédé selon l'invention tel que mis en œuvre dans l'expérience 1, dont le résultat obtenu est illustré sur la figure 1, permet, quatre fois plus rapidement (15 min versus 1 h), de purifier la protéine Mmi1 avec un degré de pureté bien plus élevé.

### C/ Expérience 3 - comparaison avec une immunoprécipitation TAP

Cette expérience est réalisée avec la protéine Mmi1 endogène de *S. pombe* fusionnée à la TAP.

Schématiquement, un extrait obtenu par lyse de cellules exprimant la protéine Mmi1 en fusion avec la protéine A est mis en présence pendant 1 h avec des billes de Sepharose^{®} sur lesquelles sont greffés des IgG, afin d'isoler la protéine Mmi1 du milieu en tirant profit de la haute affinité des IgG pour la protéine A (Kd > 10⁻⁹ M).

Après séparation des billes du milieu, les protéines fixées sur les billes sont éluées, et analysées par western blot, après séparation des protéines par électrophorèse sur gel SDS-PAGE, au moyen de l'anticorps anti-Mmi1.

Les résultats obtenus sont montrés sur la figure 4. On observe bien, sur la piste associée aux protéines capturées par immunoprécipitation avec les IgG (piste « Mmi1-TAP »), une bande correspondant au poids moléculaire de la protéine de fusion Protéine A - Mmi1.

En comparaison de cette purification par le système « Protéine A - IgG », le procédé selon l'invention tel que mis en œuvre dans l'expérience 1, dont le résultat obtenu est illustré sur la figure 1, permet, quatre fois plus rapidement (15 min versus 1 h), de purifier la protéine Mmi1 avec un degré de pureté équivalent, et même légèrement supérieur, notamment car il évite toute contamination avec les IgG.

### D/ Expérience 4 - fusion avec la protéine GFP

Cette expérience est réalisée avec la protéine Mmi1 produite dans les cellules HEK293, en fusion avec la protéine GFP.

A cet effet, le gène de séquence SEQ ID No : 2 est cloné dans le plasmide pEGFP-C3 ou pEGFP-N3, pour permettre l'expression par les cellules HEK293, de la protéine Mmi1 (complète) en fusion, en C-terminal ou en N-terminal, avec la protéine GFP. Un témoin GFP seul est également réalisé.

Les cellules obtenues sont analysées par western blot après lyse cellulaire ou observées par microscopie par fluorescence pour y vérifier la localisation de la GFP.

Les résultats obtenus sont montrés sur la figure 5 pour l'analyse par western blot après lyse cellulaire et sur la figure 6 pour l'analyse de fluorescence des cellules non lysées.

Ces résultats démontrent que la protéine de fusion de la GFP avec Mmi1 ne présente pas de toxicité pour les cellules humaines, qu'elle est bien exprimée et localisée dans les cellules. Les résultats de l'analyse par western blot confirment que les tailles des protéines détectées correspondent bien à celles qui étaient attendues (30 KDa pour la GFP seule et 84 KDa pour la protéine de fusion Mmi1-GFP), démontrant la faisabilité du procédé selon l'invention dans les cellules mammifères.

## Revendications

1. Procédé de purification d'une protéine d'intérêt, **caractérisé en ce qu'**il comprend, successivement :
- la préparation d'une protéine de fusion comprenant ladite protéine d'intérêt fusionnée à une étiquette protéique, ladite étiquette protéique comprenant au moins : la protéine Mmi1 d'un microorganisme du genre *Schizosaccharomyces,* un fragment de ladite protéine Mmi1 en comprenant au moins les 173 acides aminés C-terminaux, ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC,
- la mise en présence de ladite protéine de fusion avec une molécule d'acide ribonucléique contenant au moins un motif de séquence nucléotidique UNAAAC, de sorte à permettre la liaison par affinité de ladite étiquette protéique avec ladite molécule d'acide ribonucléique, ladite molécule d'acide ribonucléique étant greffée sur un support solide ou couplée à un ligand de capture,
- le cas échéant, la mise en présence de ladite molécule d'acide ribonucléique couplée à un ligand de capture avec un partenaire d'affinité dudit ligand de capture greffé sur un support solide,
- et la séparation de ladite protéine d'intérêt et dudit support solide.

2. Procédé de purification selon la revendication 1, selon lequel un site de clivage enzymatique est inséré entre ladite protéine d'intérêt et ladite étiquette protéique.

3. Procédé de purification selon la revendication 2, selon lequel la séparation de ladite protéine d'intérêt et dudit support solide est réalisée par clivage au niveau dudit site de clivage enzymatique.

4. Procédé de purification selon la revendication 1 ou 2, selon lequel un site de clivage est inséré entre ladite molécule d'acide ribonucléique contenant au moins un motif de séquence nucléotidique UNAAAC et ledit support solide ou ledit ligand d'intérêt, et la séparation de ladite protéine d'intérêt et dudit support solide est réalisée par clivage au niveau dudit site de clivage.

5. Procédé de purification selon l'une quelconque des revendications 1 à 4, selon lequel ledit support solide est un support de chromatographie, en particulier un polymère réticulé à base de polysaccharide ou de polyacrylamide.

6. Procédé de purification selon l'une quelconque des revendications 1 à 5, selon lequel ladite molécule d'acide ribonucléique contient une ou plusieurs répétitions dudit motif de séquence UNAAAC.

7. Procédé de purification selon l'une quelconque des revendications 1 à 6, selon lequel ladite molécule d'acide ribonucléique comprend au moins un nucléotide chimiquement modifié et/ou au moins un acide nucléique bloqué et/ou au moins une liaison internucléotidique phosphorothiate.

8. Procédé de purification selon l'une quelconque des revendications 1 à 7, selon lequel ladite protéine Mmi1 est issue d'une espèce choisie parmi *Schizosaccharomyces pombe, Schizosaccharomyces japonicus, Schizosaccharomyces octosporus* et *Schizosaccharomyces cryophilus.*

9. Procédé selon l'une quelconque des revendications 1 à 8, selon lequel ledit fragment de ladite protéine Mmi1 en comprenant au moins les 173 acides aminés C-terminaux présente une séquence d'acides aminés comprenant, ou consistant en, la séquence d'acides aminés SEQ ID No : 9, SEQ ID No : 10, SEQ ID No : 11 ou SEQ ID No : 12.

10. Procédé selon l'une quelconque des revendications 1 à 9, selon lequel ladite étiquette protéique contient au moins un domaine choisi parmi les domaines de séquences d'acides aminés :
- RSVWXaa₁Xaa₂Xaa₃Xaa₄Xaa₅Xaa₆P (SEQ ID No : 13), où Xaa₁ représente un résidu thréonine, sérine ou alanine, Xaa₂ représente un résidu thréonine, arginine, lysine ou sérine, Xaa₃ représente un résidu histidine ou arginine, Xaa₄ représente un résidu thréonine ou proline, Xaa₅ représente un résidu glycine, alanine ou arginine et Xaa₆ représente un résidu acide glutamique ou acide aspartique,
- FXaa₇SPLKRXaa₈APXaa₉SXaa₁₀Xaa₁₁Xaa₁₂Xaa₁₃Xaa₁₄Xaa₁₅R (SEQ ID No : 14), où Xaa₇ représente un résidu sérine ou thréonine, Xaa₈ représente un résidu proline ou glycine, Xaa₉ représente un résidu acide glutamique ou acide aspartique, Xaa₁₀ représente un résidu histidine, arginine ou lysine, Xaa₁₁ représente un résidu acide aspartique ou acide glutamique, Xaa₁₂ représente un résidu alanine ou tyrosine, Xaa₁₃ est nul ou représente un résidu proline, Xaa₁₄ représente un résidu isoleucine ou méthionine et Xaa₁₅ représente un résidu glycine ou acide aspartique,
- YDFXaa₁₆RHCTDYGHSYXaa₁₇WPYFRSXaa₁₈RREXaa₁₉Xaa₂₀Xaa₂₁Y (SEQ ID No : 15), où Xaa₁₆ représente un résidu sérine, thréonine ou tyrosine, Xaa₁₇ représente un résidu acide glutamique ou acide aspartique, Xaa₁₈ représente un résidu leucine ou valine, Xaa₁₉ est nul ou représente un résidu sérine, Xaa₂₀ représente un résidu leucine ou méthionine et Xaa₂₁ représente un résidu arginine, leucine ou méthionine,
- QPPXaa₂₂KRRTLXaa₂₃Xaa₂₄P (SEQ ID No : 16), où Xaa₂₂ représente un résidu proline, sérine ou leucine, Xaa₂₃ représente un résidu sérine ou leucine et Xaa₂₄ représente un résidu proline ou sérine,
- Xaa₂₅AXaa₂₆Xaa₂₇SPXaa₂₈Xaa₂₉Xaa₃₀Xaa₃₁PXaa₃₂Xaa₃₃H (SEQ ID No : 17), où Xaa₂₅ représente un résidu arginine ou acide aspartique, Xaa₂₆ représente un résidu sérine ou glycine, Xaa₂₇ représente un résidu histidine ou acide aspartique, Xaa₂₈ représente un résidu sérine, glycine ou leucine, Xaa₂₉ représente un résidu leucine ou phénylalanine, Xaa₃₀ représente un résidu leucine, isoleucine ou sérine, Xaa₃₁ représente un résidu acide glutamique ou acide aspartique, Xaa₃₂ représente un résidu tyrosine ou thréonine et Xaa₃₃ représente un résidu alanine ou thréonine,
- RXaa₃₄EKPKXaa₃₅RAXaa₃₆TPPP (SEQ ID No : 18), où Xaa₃₄ représente un résidu lysine ou arginine, Xaa₃₅ représente un résidu alanine, proline ou thréonine et Xaa₃₆ représente un résidu sérine ou proline.

11. Support solide pour la mise en œuvre d'un procédé de purification d'une protéine d'intérêt selon l'une quelconque des revendications 1 à 10, **caractérisé en ce qu'**une molécule d'acide ribonucléique contenant au moins un motif de séquence nucléotidique UNAAAC est greffée sur ledit support solide.

12. Kit pour la mise en œuvre d'un procédé de purification d'une protéine d'intérêt selon l'une quelconque des revendications 1 à 10, **caractérisé en ce qu'**il contient une molécule d'acide ribonucléique contenant au moins un motif de séquence nucléotidique UNAAAC greffée sur un support solide ou couplée à un ligand de capture, et au moins un des constituants suivants :
- un vecteur d'expression comprenant, sous le contrôle d'un promoteur, une molécule d'acide nucléique codant pour une étiquette protéique comprenant au moins : la protéine Mmi1 d'un microorganisme du genre *Schizosaccharomyces,* un fragment de ladite protéine Mmi1 en comprenant au moins les 173 acides aminés C-terminaux, ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC ; et un site permettant l'insertion, en 5' ou en 3' par rapport à ladite molécule d'acide nucléique codant pour ladite étiquette protéique, d'une molécule d'acide nucléique codant pour ladite protéine d'intérêt de sorte à permettre l'expression d'une protéine de fusion contenant ladite protéine d'intérêt et ladite étiquette protéique ;
- des instructions pour la mise en œuvre des étapes d'un procédé selon l'une quelconque des revendications 1 à 10.

13. Kit selon la revendication 12, contenant en outre une cellule hôte apte à être transformée par un vecteur d'expression comprenant, sous le contrôle d'un promoteur, une molécule d'acide nucléique codant pour une étiquette protéique comprenant au moins : la protéine Mmi1 d'un microorganisme du genre *Schizosaccharomyces,* un fragment de ladite protéine Mmi1 en comprenant au moins les 173 acides aminés C-terminaux, ou une protéine de séquence d'acides aminés ayant au moins 90 % d'identité avec la séquence d'acides aminés de ladite protéine Mmi1 ou dudit fragment et capable de se lier à un motif d'acide ribonucléique de séquence nucléotidique UNAAAC ; et un site permettant l'insertion, en 5' ou en 3' par rapport à ladite molécule d'acide nucléique codant pour ladite étiquette protéique, d'une molécule d'acide nucléique codant pour ladite protéine d'intérêt de sorte à permettre l'expression d'une protéine de fusion contenant ladite protéine d'intérêt et ladite étiquette protéique.

## Patentansprüche

1. Reinigungsverfahren eines Proteins von Interesse, **dadurch gekennzeichnet, dass** es nacheinander Folgendes umfasst:
- Herstellen eines Fusionsproteins, das das Protein von Interesse umfasst, das mit einem Protein-Tag zusammengefügt ist, wobei das Protein-Tag mindestens Folgendes umfasst: das Mmi1-Protein eines Mikroorganismus der Gattung *Schizosaccharomyces,* ein Fragment des Mmi1-Proteins, das mindestens die 173 C-terminalen Aminosäuren umfasst, oder ein Aminosäuresequenzprotein, das mindestens 90 % mit der Aminosäuresequenz des Mmi1-Proteins oder des Fragments identisch ist und dazu ausgelegt ist, sich an ein UNAAAC-Nukleotidsequenz-Ribonukleinsäuremuster zu binden,
- Zusammenbringen des Fusionsproteins mit einem Ribonukleinsäuremolekül, das mindestens ein UNAAAC-Nukleotidsequenzmuster enthält, so dass eine Affinitätsbindung des Protein-Tags mit dem Ribonukleinsäuremolekül ermöglicht wird, wobei das Ribonukleinsäuremolekül auf einen festen Träger transplantiert oder mit einem Fang-Ligand gekoppelt ist,
- gegebenenfalls Zusammenbringen des Ribonukleinsäuremoleküls, das mit einem Fang-Ligand gekoppelt ist, mit einem Affinitätspartner des Fang-Ligands, der auf einen festen Träger transplantiert ist,
- und Trennen des Proteins von Interesse und des festen Trägers.

2. Reinigungsverfahren nach Anspruch 1, wobei eine enzymatische Spaltstelle zwischen dem Protein von Interesse und dem Protein-Tag eingefügt wird.

3. Reinigungsverfahren nach Anspruch 2, wobei die Trennung des Proteins von Interesse und des festen Trägers durch Spaltung an der enzymatischen Spaltstelle erfolgt.

4. Reinigungsverfahren nach Anspruch 1 oder 2, wobei eine Spaltstelle zwischen dem Ribonukleinsäuremolekül, das mindestens ein UNAAAC-Nukleotidsequenzmuster enthält, und dem festen Träger oder Ligand von Interesse eingefügt wird, und die Trennung des Proteins von Interesse und des festen Trägers durch Spaltung an der Spaltstelle erfolgt.

5. Reinigungsverfahren nach einem der Ansprüche 1 bis 4, wobei der feste Träger ein Chromatographieträger ist, insbesondere ein vernetztes Polymer auf Polysaccharid- oder Polyacrylamidbasis.

6. Reinigungsverfahren nach einem der Ansprüche 1 bis 5, wobei das Ribonukleinsäuremolekül eine oder mehrere Wiederholungen des UNAAAC-Sequenzmusters enthält.

7. Reinigungsverfahren nach einem der Ansprüche 1 bis 6, wobei das Ribonukleinsäuremolekül mindestens ein chemisch modifiziertes Nukleotid und/oder mindestens eine blockierte Nukleinsäure und/oder mindestens eine Phosphorrothiat-Internukleotid-Bindung umfasst.

8. Reinigungsverfahren nach einem der Ansprüche 1 bis 7, wobei das Mmi1-Protein aus einer Art stammt, die unter *Schizosaccharomyces pombe, Schizosaccharomyces japonicus, Schizosaccharomyces octosporus* und *Schizosaccharomyces cryophilus* ausgewählt ist.

9. Verfahren nach einem der Ansprüche 1 bis 8, wobei das Fragment des Mmi1-Proteins, das mindestens die 173 C-terminalen Aminosäuren umfasst, eine Aminosäuresequenz besitzt, die die Aminosäuresequenz SEQ ID-Nr.: 9, SEQ ID-Nr.: 10, SEQ ID-Nr.: 11 oder SEQ ID-Nr.: 12 umfasst.

10. Verfahren nach einem der Ansprüche 1 bis 9, wobei das Protein-Tag mindestens einen Bereich enthält, der aus den Aminosäuresequenzbereichen ausgewählt ist:
- RSVWXaa₁Xaa₂Xaa₃Xaa₄Xaa₅Xaa₆P (SEQ ID Nr.: 13), wobei Xaa₁ einen Threonin-, Serin- oder Alaninrest darstellt, Xaa₂ einen Threonin-, Arginin-, Lysin- oder Serinrest darstellt, Xaa₃ einen Histidin- oder Argininrest darstellt, Xaa₄ einen Threonin- oder Prolinrest darstellt, Xaa₅ einen Glycin-, Alanin- oder Argininrest darstellt und Xaa₆ einen Glutaminsäure- oder Asparaginsäurerest darstellt,
- FXaa₇SPLKRXaa₈APXaa₉SXaa₁₀Xaa₁₁Xaa₁₂Xaa₁₃Xaa₁₄Xaa₁₅R (SEQ ID Nr.: 14), wobei Xaa₇ einen Serin- oder Threoninrest darstellt, Xaa₈ einen Prolin- oder Glycinrest darstellt, Xaa₉ einen Glutaminsäure- oder Asparaginsäurerest darstellt, Xaa₁₀ einen Histidin-, Arginin- oder Lysinrest darstellt, Xaa₁₁ einen Asparaginsäure- oder Glutaminsäurerest darstellt, Xaa₁₂ einen Alanin- oder Tyrosinrest darstellt, Xaa₁₃ null ist oder einen Prolinrest darstellt, Xaa₁₄ einen Isoleucin- oder Methioninrest darstellt und Xaa₁₅ einen Glycin- oder Asparaginsäurerest darstellt,
- YDFXaa₁₆RHCTDYGHSYXaa₁₇WPYFRSXaa₁₈RREXaa₁₉Xaa₂₀Xaa₂₁Y (SEQ ID Nr.: 15), wobei Xaa₁₆ einen Serin-, Threonin- oder Tyrosinrest darstellt, Xaa₁₇ einen Glutaminsäure- oder Asparaginsäurerest darstellt, Xaa₁₈ einen Leucin- oder Valinrest darstellt, Xaa₁₉ null ist oder einen Serinrest darstellt, Xaa₂₀ einen Leucin- oder Methioninrest darstellt und Xaa₂₁ einen Arginin-, Leucin- oder Methioninrest darstellt,
- QPPXaa₂₂KRRTLXaa₂₃Xaa₂₄P (SEQ ID Nr.: 16), wobei Xaa₂₂ einen Prolin-, Serin- oder Leucinrest darstellt, Xaa₂₃ einen Serin- oder Leucinrest darstellt und Xaa₂₄ einen Prolin- oder Serinrest darstellt,
- Xaa₂₅AXaa₂₆Xaa₂₇SPXaa₂₈Xaa₂₉Xaa₃₀Xaa₃₁PXaa₃₂Xaa₃₃H (SEQ ID Nr.: 17), wobei Xaa₂₅ einen Arginin- oder Asparaginsäurerest darstellt, Xaa₂₆ einen Serin- oder Glycinrest darstellt, Xaa₂₇ einen Histidin- oder Asparaginsäurerest darstellt, Xaa₂₈ einen Serin-, Glycin- oder Leucinrest darstellt, Xaa₂₉ einen Leucin- oder Phenylalaninrest darstellt, Xaa₃₀ einen Leucin-, Isoleucin- oder Serinrest darstellt, Xaa₃₁ einen Glutaminsäure- oder Asparaginsäurerest darstellt, Xaa₃₂ einen Tyrosin- oder Threoninrest darstellt und Xaa₃₃ einen Alanin- oder Threoninrest darstellt,
- RXaa₃₄EKPKXaa₃₅RAXaa₃₆TPPP (SEQ ID Nr.: 18), wobei Xaa₃₄ einen Lysin- oder Argininrest darstellt, Xaa₃₅ einen Alanin-, Prolin- oder Threoninrest darstellt und Xaa₃₆ einen Serin- oder Prolinrest darstellt.

11. Fester Träger für die Durchführung eines Verfahrens zur Reinigung eines Proteins von Interesse nach einem der Ansprüche 1 bis 10, **dadurch gekennzeichnet, dass** ein Ribonukleinsäuremolekül, das mindestens ein UNAAAC-Nukleotidsequenzmuster enthält, auf den festen Träger transplantiert ist.

12. Kit zur Durchführung eines Verfahrens zur Reinigung eines Proteins von Interesse nach einem der Ansprüche 1 bis 10, **dadurch gekennzeichnet, dass** es ein Ribonukleinsäuremolekül, das mindestens ein UNAAAC-Nukleotidsequenzmuster enthält, das auf einem festen Träger transplantiert oder mit einem Fang-Ligand gekoppelt ist, und mindestens einen der folgenden Bestandteile enthält:
- einen Expressionsvektor, der unter der Kontrolle eines Promotors ein Nukleinsäuremolekül umfasst, das für ein Protein-Tag kodiert, das mindestens Folgendes umfasst: das Mmi1-Protein eines Mikroorganismus der Gattung *Schizosaccharomyces,* ein Fragment des Mmi1-Proteins, das mindestens die 173 C-terminalen Aminosäuren umfasst, oder ein Aminosäuresequenzprotein, das mindestens 90 % mit der Aminosäuresequenz des Mmi1-Proteins oder des Fragments identisch ist und in der Lage ist, an ein UNAAAC-Nukleotidsequenz-Ribonukleinsäuremuster zu binden; und eine Stelle, an der ein Nukleinsäuremolekül, das für das Protein-Tag kodiert, in Position 5' oder 3' in Bezug auf das Nukleinsäuremolekül, das für das Protein von Interesse kodiert, eingefügt werden kann, so dass ein Fusionsprotein, das das Protein von Interesse und das Protein-Tag enthält, exprimiert werden kann;
- Anweisungen zur Durchführung der Schritte eines Verfahrens nach einem der Ansprüche 1 bis 10.

13. Kit nach Anspruch 12, ferner enthaltend eine Wirtszelle, die durch einen Expressionsvektor transformierbar ist, der unter der Kontrolle eines Promotors ein Nukleinsäuremolekül umfasst, das für ein Protein-Tag kodiert, das mindestens Folgendes umfasst: das Mmi1-Protein eines Mikroorganismus der Gattung *Schizosaccharomyces,* ein Fragment des Mmi1-Proteins, das mindestens die 173 C-terminalen Aminosäuren umfasst, oder ein Aminosäuresequenzprotein, das mindestens 90 % mit der Aminosäuresequenz des Mmi1-Proteins oder des Fragments identisch ist und in der Lage ist, an ein UNAAAC-Nukleotidsequenz-Ribonukleinsäuremuster zu binden; und eine Stelle, an der ein Nukleinsäuremolekül, das für das Protein-Tag kodiert, in Position 5' oder 3' in Bezug auf das Nukleinsäuremolekül, das für das Protein von Interesse kodiert, eingefügt werden kann, so dass ein Fusionsprotein, das das Protein von Interesse und das Protein-Tag enthält, exprimiert werden kann.

## Claims

1. A method for purifying a protein of interest, **characterized in that** it successively comprises:
- the preparation of a fusion protein comprising said protein of interest fused to a protein tag, said protein tag comprising at least: the protein Mmi1 of a microorganism of the genus *Schizosaccharomyces,* a fragment of said protein Mmi1 comprising at least the 173 C-terminal amino acids, or a protein of amino acid sequence having at least 90% identity with the amino acid sequence of said protein Mmi1 or of said fragment and capable of binding to a ribonucleic acid motif of UNAAAC nucleotide sequence,
- the bringing together of said fusion protein with a ribonucleic acid molecule containing at least one motif of UNAAAC nucleotide sequence, so as to allow the affinity binding of said protein tag with said ribonucleic acid molecule, said ribonucleic acid molecule being grafted onto a solid support or coupled to a capture ligand,
- where appropriate, the bringing together of said ribonucleic acid molecule coupled to a capture ligand with an affinity partner of said capture ligand grafted onto a solid support,
- and the separation of said protein of interest and said solid support.

2. The purification method according to claim 1, according to which an enzymatic cleavage site is inserted between said protein of interest and said protein tag.

3. The purification method according to claim 2, according to which the separation of said protein of interest and said solid support is achieved by cleavage at said enzymatic cleavage site.

4. The purification method according to claim 1 or 2, according to which a cleavage site is inserted between said ribonucleic acid molecule containing at least one motif of UNAAAC nucleotide sequence and said solid support or said ligand of interest, and the separation of said protein of interest and said solid support is achieved by cleavage at said cleavage site.

5. The purification method according to any one of claims 1 to 4, according to which said solid support is a chromatography support, in particular a polysaccharide or polyacrylamide-based crosslinked polymer.

6. The purification method according to any one of claims 1 to 5, according to which said ribonucleic acid molecule contains one or several repeats of said motif of UNAAAC sequence.

7. The purification method according to any one of claims 1 to 6, according to which said ribonucleic acid molecule comprises at least one chemically modified nucleotide and/or at least one locked nucleic acid and/or at least one phosphorothioate internucleotide bond.

8. The purification method according to any one of claims 1 to 7, according to which said protein Mmi1 is derived from a species selected from *Schizosaccharomyces pombe, Schizosaccharomyces japonicus, Schizosaccharomyces octosporus* and *Schizosaccharomyces cryophilus.*

9. The method according to any one of claims 1 to 8, according to which said fragment of said protein Mmi1 comprising at least the 173 C-terminal amino acids has an amino acid sequence comprising, or consisting of, the amino acid sequence SEQ ID No: 9, SEQ ID No: 10, SEQ ID No: 11 or SEQ ID No: 12.

10. The method according to any one of claims 1 to 9, according to which said protein tag contains at least one domain selected from the domains of amino acid sequences:
- RSVWXaa₁Xaa₂Xaa₃Xaa₄Xaa₅Xaa₆P (SEQ ID No: 13), where Xaa₁ represents a threonine, serine or alanine residue, Xaa₂ represents a threonine, arginine, lysine or serine residue, Xaa₃ represents a histidine or arginine residue, Xaa₄ represents a threonine or proline residue, Xaa₅ represents a glycine, alanine or arginine residue and Xaa₆ represents a glutamic acid or aspartic acid residue,
- FXaa₇SPLKRXaa₈APXaa₉SXaa₁₀Xaa₁₁Xaa₁₂Xaa₁₃Xaa₁₄Xaa₁₅R (SEQ ID No: 14), where Xaa₇ represents a serine or threonine residue, Xaa₈ represents a proline or glycine residue, Xaa₉ represents a glutamic acid or aspartic acid residue, Xaa₁₀ represents a histidine, arginine or lysine residue, Xaa₁₁ represents an aspartic acid or glutamic acid residue, Xaa₁₂ represents an alanine or tyrosine residue, Xaa₁₃ is zero or represents a proline residue, Xaa₁₄ represents an isoleucine or methionine residue and Xaa₁₅ represents a glycine or aspartic acid residue,
- YDFXaa₁₆RHCTDYGHSYXaa₁₇WPYFRSXaa₁₈RREXaa₁₉Xaa₂₀Xaa₂₁Y (SEQ ID No: 15), where Xaa₁₆ represents a serine, threonine or tyrosine residue, Xaa₁₇ represents a glutamic acid or aspartic acid residue, Xaa₁₈ represents a leucine or valine residue, Xaa₁₉ is zero or represents a serine residue, Xaa₂₀ represents a leucine or methionine residue and Xaa₂₁ represents an arginine, leucine or methionine residue,
- QPPXaa₂₂KRRTLXaa₂₃Xaa₂₄P (SEQ ID No: 16), where Xaa₂₂ represents a proline, serine or leucine residue, Xaa₂₃ represents a serine or leucine residue and Xaa₂₄ represents a proline or serine residue,
- Xaa₂₅AXaa₂₆Xaa₂₇SPXaa₂₈Xaa₂₉Xaa₃₀Xaa₃₁PXaa₃₂Xaa₃₃H (SEQ ID No: 17), where Xaa₂₅ represents an arginine or aspartic acid residue, Xaa₂₆ represents a serine or glycine residue, Xaa₂₇ represents a histidine or aspartic acid residue, Xaa₂₈ represents a serine, glycine or leucine residue, Xaa₂₉ represents a leucine or phenylalanine residue, Xaa₃₀ represents a leucine, isoleucine or serine residue, Xaa₃₁ represents a glutamic acid or aspartic acid residue, Xaa₃₂ represents a tyrosine or threonine residue and Xaa₃₃ represents an alanine or threonine residue,
- RXaa₃₄EKPKXaa₃₅RAXaa₃₆TPPP (SEQ ID No: 18), where Xaa₃₄ represents a lysine or arginine residue, Xaa₃₅ represents an alanine, proline or threonine residue and Xaa₃₆ represents a serine or proline residue.

11. A solid support for implementing a method for purifying a protein of interest according to any one of claims 1 to 10, **characterized in that** a ribonucleic acid molecule containing at least one motif of UNAAAC nucleotide sequence is grafted onto said solid support.

12. A kit for implementing a method for purifying a protein of interest according to any one of claims 1 to 10, **characterized in that** it contains a ribonucleic acid molecule containing at least one motif of UNAAAC nucleotide sequence grafted onto a solid support or coupled to a capture ligand, and at least one of the following constituents:
- an expression vector comprising, under the control of a promoter, a nucleic acid molecule encoding a protein tag comprising at least: the protein Mmi1 of a microorganism of the genus *Schizosaccharomyces,* a fragment of said protein Mmi1 comprising at least the 173 C-terminal amino acids, or a protein of amino acid sequence having at least 90% identity with the amino acid sequence of said protein Mmi1 or of said fragment and capable of binding to a ribonucleic acid motif of UNAAAC nucleotide sequence; and a site allowing the insertion, at 5' or at 3' relative to said nucleic acid molecule encoding said protein tag, of a nucleic acid molecule encoding said protein of interest so as to allow the expression of a fusion protein containing said protein of interest and said protein tag;
- instructions for implementing the steps of a method according to any one of claims 1 to 10.

13. The kit according to claim 12, further containing a host cell capable of being transformed by an expression vector comprising, under the control of a promoter, a nucleic acid molecule encoding a protein tag comprising at least: the protein Mmi1 of a microorganism of the genus *Schizosaccharomyces, a* fragment of said protein Mmi1 comprising at least the 173 C-terminal amino acids, or a protein of amino acid sequence having at least 90% identity with the amino acid sequence of said protein Mmi1 or of said fragment and capable of binding to a ribonucleic acid motif of UNAAAC nucleotide sequence; and a site allowing the insertion, at 5' or at 3' relative to said nucleic acid molecule encoding said protein tag, of a nucleic acid molecule encoding said protein of interest so as to allow the expression of a fusion protein containing said protein of interest and said protein tag.
